# EUROPÄISCHE PATENTSCHRIFT

(11) **EP 3 100 694 B1**
(45) Veröffentlichungstag und Bekanntmachung des Hinweises auf die Patenterteilung: **11.03.2020**
(21) Anmeldenummer: 16169158.9
(22) Anmeldetag: 11.05.2016
(51) Int. Cl.: A61B 17/88, B01F 13/06, B01F 13/00, B01F 15/02, B01F 11/00

(54) **VORRICHTUNG UND VERFAHREN ZUM MISCHEN UND LAGERN VON POLYMETHYLMETHACRYLAT-KNOCHENZEMENT**
DEVICE AND METHOD FOR MIXING AND STORING POLYMETHYMETHACRYLATE BONE CEMENT
DISPOSITIF ET PROCÉDÉ DE MÉLANGE ET DE STOCKAGE DE CIMENT OSSEUX EN POLYMÉTHACRYLATE DE MÉTHYLE

(30) Priorität: 03.06.2015 DE 102015108783
(43) Veröffentlichungstag der Anmeldung: 07.12.2016
(73) Patentinhaber: Heraeus Medical GmbH, 61273 Wehrheim (DE)
(72) Erfinder: Dr. Vogt, Sebastian, 99092 Erfurt (DE); Dr. Kluge, Thomas, 56179 Vallendar (DE)
(74) Vertreter: Sterzel, Roland

(56) Entgegenhaltungen:
- EP-A1- 2 957 337
- DE-B3-102009 031 178
- DE-U1- 20 005 333

## Beschreibung

Die Erfindung betrifft ein Mischsystem zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten.

Die Erfindung betrifft ferner ein Verfahren zum Mischen von Polymethylmethacrylat-Knochenzement.

Gegenstand der Erfindung ist somit eine Vorrichtung zur Lagerung und Vermischung von Polymethylmethacrylat-Knochenzement sowie Verfahren zum Mischen von Polymethylmethacrylat-Knochenzement.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig entstehen können beziehungsweise vorhanden sein können, welche die mechanischen Eigenschaften des ausgehärteten Knochenzements negativ beeinflussen können und die somit später eine Destabilisierung des Knochenzements verursachen können.

Zur Vermeidung von Lufteinschlüssen im Knochenzementteig wurden eine Vielzahl von Vakuum-Zementiersystemen vorgeschlagen, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig. Eine Vorrichtung zum Mischen von Knochenzement, bei der das Vakuum mit einer Handpumpe erzeugt werden kann und bei der eine Monomer-Flüssigkeit mit einer anschließbaren Spritze injiziert wird, ist aus der EP 1 259 200 B1 bekannt. Das dort beschriebene System ist jedoch kein Full-Prepacked-Mischsystem und hat zusätzlich den Nachteil, dass die Vakuumpumpe und die Spritze mit der Monomerflüssigkeit aufwendig manuell mit der Vorrichtung verbunden werden müssen. Durch die verschiedenen notwendigen Handgriffe ist die Handhabung der Vorrichtung eher aufwendig.

Das Patent DE 10 2009 031 178 B3 offenbart eine gattungsgemäße Mischvorrichtung mit einem zweiteiligen Austragskolben zum Verschluss einer Zementkartusche. Dabei wird eine Kombination aus einem Gas-durchlässigen Sterilisationskolben und einem Gas-undurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei der Verwendung von Vakuummischsystemen zur Zementierung müssen meist externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für eine Mischstange oder ein Mischrohr nicht ohne weiteres dauerhaft vakuumdicht ist.

DE 200 05 333 U1 offenbart eine Vorrichtung entsprechend dem Oberbegriff des Anspruch 1 und beschreibt eine Applikationsvorrichtung für Implantatmaterialien mit zwei Behältern, von denen ein erster eine pulverförmige und ein zweiter eine flüssige Komponente enthalten. Zwischen den Behältern sind Mittel zur Herstellung einer Oberströmverbindung vorgsehen. Der erste Behälter enthält ferner einen hohlen Mischschaft, der an seinem äußeren Ende an eine Handpumpe angeschlossen ist, mit der Unterdruck erzeugt werden kann.

Die Aufgabe der Erfindung besteht also darin, die Nachteile des Stands der Technik zu überwinden. Insbesondere sollen die Nachteile der bekannten Vakuummischsysteme mit externer Vakuumquelle und/oder aufwendigem Aufbau und aufwendiger Bedienung überwunden werden. Die Erfindung hat dabei unter anderem die Aufgabe, eine einfache, geschlossene Vorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver (PMMA-Knochenzement-Pulver) mit der Monomerflüssigkeit innerhalb der Vorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich um ein Full-Prepacked-Mischsystem. Die Vorrichtung soll so beschaffen sein, dass ein Transfer der Monomerflüssigkeit in das Polymethylmethacrylat-Knochenzementpulver ohne die Verwendung von durch Druckluft oder Kompressoren angetriebenen externen Vakuumpumpen, transferiert werden kann. Wichtig ist ferner, dass die Vorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Vorrichtung soll ohne zusätzliche technische Ausrüstung autonom verwendungsfähig sein. Der Aufbau soll ferner möglichst einfach und kostengünstig sein. Die Vorrichtung soll auch unter schwierigen Bedingungen einfach zu bedienen sein.

Weiterhin hat die Erfindung die Aufgabe, eine Vorrichtung bereit zu stellen, bei der es möglich ist, dass Volumen an Monomerflüssigkeit, die in das Zementpulver transferiert wird, gezielt zu steuern, so dass das Verhältnis des Volumens an Monomerflüssigkeit zur Zementpulvermenge variiert werden kann, um die Konsistenz und damit die Verarbeitungseigenschaften des Knochenzements zu steuern. Es sollen ferner Luftblasen im fertig gemischten Knochenzement vermieden werden.

Es soll ferner ein Verfahren bereitgestellt werden, das einen Monomertransfer und ein Mischen in Full-Prepacked-Mischsystemen ermöglicht. Dabei soll das zu entwickelnde Mischsystem (die Vorrichtung), hauptsächlich aus kostengünstigem Kunststoff gefertigt werden können.

Des Weiteren soll eine kostengünstig zu fertigende und zuverlässig funktionierende Vorrichtung zum Mischen eines medizinischen Zements und gegebenenfalls zur Lagerung der Ausgangskomponenten des Zements sowie ein Verfahren zum Mischen des Knochenzements gefunden werden, bei dem eine möglichst einfache manuelle Bedienung zum Mischen der Ausgangskomponenten angewendet werden kann, möglichst ohne dass eine externe oder zusätzliche Energiequelle verwendet werden muss und ohne dass Lufteinschlüsse in dem Mischgut entstehen.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in dem Full-Prepacked-Mischsystem getrennt gelagert werden können und durch Anwendung der Vorrichtung sicher zusammengeführt werden können.

Die Aufgaben der Erfindung werden gelöst durch eine Vorrichtung zum Mischen von Polymethylmethacrylat-Knochenzement und zum Lagern der Ausgangskomponenten des Knochenzements, insbesondere einer Monomerflüssigkeit und eines Zementpulvers als Ausgangskomponenten des Knochenzements, die Vorrichtung aufweisend
A) eine Kartusche mit einem Innenraum zum Mischen des Knochenzements, der auf einer Seite durch einen beweglichen Austragskolben verschlossen ist,
B) einen Monomer-Behälter für eine Monomerflüssigkeit und/oder einen Anschluss zur Befestigung eines Monomer-Behälters für eine Monomerflüssigkeit, so dass der Monomer-Behälter in der Vorrichtung derart zu öffnen ist, dass die Monomerflüssigkeit aus dem Monomer-Behälter in die Vorrichtung ausläuft,
C) eine Verbindungsleitung, durch die die Monomerflüssigkeit in den Innenraum der Kartusche zu leiten ist, wobei
D) ein erster Hohlzylinder mit der Verbindungsleitung verbunden ist und ein zweiter Hohlzylinder über eine Vakuumleitung mit dem Innenraum der Kartusche verbunden ist, wobei in dem ersten Hohlzylinder ein axial im ersten Hohlzylinder verschiebbarer Pumpkolben angeordnet ist und in dem zweiten Hohlzylinder ein axial im zweiten Hohlzylinder verschiebbarer Vakuumkolben angeordnet ist, wobei vorgesehen ist, dass der Pumpkolben und der Vakuumkolben zeitgleich bewegbar sind.

Insbesondere können der Pumpkolben und der Vakuumkolben synchron miteinander bewegbar sein.

Der erste Hohlzylinder ist derart mit dem Monomer-Behälter und/oder dem Anschluss zur Befestigung eines Monomer-Behälters verbunden, dass die Monomerflüssigkeit aus dem geöffneten Monomer-Behälter oder aus einem geöffneten angeschlossenen Monomer-Behälter in den ersten Hohlzylinder fließt und die Verbindungsleitung den ersten Hohlzylinder mit dem Innenraum der Kartusche derart verbindet, dass mit dem Pumpkolben Monomerflüssigkeit aus dem ersten Hohlzylinder durch Betätigen des Pumpkolbens durch die Verbindungsleitung in den Innenraum der Kartusche zu drücken ist.

Der erste und der zweite Hohlzylinder können besonders bevorzugt auch als gemeinsamer Hohlzylinder ausgeführt sein, in dem sich ein kombinierter Pump-Vakuum-Kolben bewegt, wobei eine erste Seite, insbesondere die Unterseite, des Pump-Vakuum-Kolbens den Pumpkolben bildet und eine zweite Seite, insbesondere die Oberseite, des Pump-Vakuum-Kolbens den Vakuumkolben. Der obere Teil des gemeinsamen Hohlzylinders ist dann im Rahmen der vorliegenden Erfindung als der zweite Hohlzylinder und der untere Teil des gemeinsamen Hohlzylinders als der erste Hohlzylinder aufzufassen.

Damit die Monomerflüssigkeit aus dem geöffneten Monomer-Behälter in den ersten Hohlzylinder fließt, ist der geöffnete Monomer-Behälter hierzu mit dem ersten Hohlzy linder verbunden, vorzugsweise über eine Einmündung mit dem ersten Hohlzylinder verbunden.

Damit die Monomerflüssigkeit ohne zusätzliche Krafteinwirkung fließen kann, muss die Vorrichtung bestimmungsgemäß aufgestellt werden, damit die Gravitation die gewünschte Flussrichtung bewirkt. Demzufolge sind die im Rahmen der vorliegenden Erfindung verwendeten Begriffe oben und unten sowie oberhalb und unterhalb und höchsten und tiefsten immer in Bezug auf die bestimmungsgemäße Aufstellung der Vorrichtung bezogen.

Der Innenraum der Kartusche hat vorzugsweise eine zylindrische Geometrie. Die zylindrische Form ist die einfachste, mit der sich der Innenraum der Kartusche und der erste und der zweite Hohlzylinder realisieren lassen. Unter einer zylindrischen Form ist geometrisch die Form eines allgemeinen Zylinders mit einer beliebigen Grundfläche zu verstehen, also nicht nur einer mit einer kreisförmigen Grundfläche. Die Innenwand des Innenraums kann also ein Zylinder mit beliebiger Grundfläche sein und der Mantel des ersten und zweiten Hohlzylinders kann ein Zylinder mit beliebiger Grundfläche sein, das heißt auch mit nicht kreisförmiger oder runder Grundfläche. Erfindungsgemäß wird jedoch eine zylindrische Geometrie mit rotationssymmetrischer Grundfläche bevorzugt.

Um eine gute Pumpwirkung zu erreichen und einen Austritt von Monomerflüssigkeit aus dem Pumpkolben beziehungsweise dem ersten Hohlzylinder zu vermeiden, schließt der Pumpkolben fluiddicht mit den Innenwänden des ersten Hohlzylinders ab. Hierzu kann eine umlaufende Dichtung vorgesehen sein, die den Pumpkolben mit den Innenwänden des ersten Hohlzylinders abschließt. Ebenso kann eine umlaufende Dichtung vorgesehen sein, um den Vakuumkolben mit den Innenwänden des zweiten Hohlzylinders abzudichten, so dass beim Erzeugen eines Unterdrucks mit dem Vakuumkolben keine oder zumindest nur sehr wenig Luft von der anderen Seite des Vakuumkolbens nachgezogen wird.

Es ist erfindungsgemäß bevorzugt, wenn der Monomer-Behälter an den Anschluss für den Monomer-Behälter angeschlossen ist oder der Monomer-Behälter in den Anschluss für den Monomer-Behälter eingesetzt ist.

Der Polymethylmethacrylat-Knochenzement wird bevorzugt aus zumindest zwei Komponenten gemischt, beziehungsweise ist aus zumindest zwei Komponenten herstellbar. Besonders bevorzugt ist eine Komponente flüssig (die Monomerflüssigkeit) und die andere Komponente pulverförmig (das Zementpulver).

Die Ausgangskomponenten für das Mischgut, insbesondere für den PMMA-Knochenzement, sind erfindungsgemäß bereits in der Kartusche und dem Monomer-Behälter enthalten.

Die Vorrichtung ist erfindungsgemäß bevorzugt auch zum Lagern der Ausgangskomponenten geeignet, insbesondere dann, wenn die Behälter, insbesondere der Monomer-Behälter, in die Vorrichtung eingesetzt sind oder die Behälter, insbesondere der Monomer-Behälter, ein fester Teil der Vorrichtung sind.

Mit der erfindungsgemäßen Vorrichtung kann auch vorgesehen sein, dass der Pumpkolben und der Vakuumkolben derart miteinander verbunden sind oder derart über ein gemeinsames Bedienelement bewegt werden, dass sich beim Einschieben des Pumpkolbens in den ersten Hohlzylinder der Vakuumkolben im zweiten Hohlzylinder von der Verbindung zur Vakuumleitung weg bewegt, wobei vorzugsweise sich dabei das Volumen zwischen dem Vakuumkolben und der Verbindung zur Vakuumleitung im zweiten Hohlzylinder vergrößert, so dass durch den im zweiten Hohlzylinder entstehenden Unterdruck ein Gas aus dem Innenraum der Kartusche durch die Vakuumleitung in den zweiten Hohlzylinder strömt.

Bei der Vergrößerung des Volumens zwischen dem Vakuumkolben und der Verbindung zur Vakuumleitung im zweiten Hohlzylinder entsteht ein Unterdruck, der Gas aus dem Innenraum der Kartusche durch die Vakuumleitung in den zweiten Hohlzylinder zieht, beziehungsweise so dass Gas aus dem Innenraum der Kartusche durch die Vakuumleitung in den zweiten Hohlzylinder gedrückt wird.

Hierdurch wird erreicht, dass mit einer einzigen Bewegung sowohl die Monomerflüssigkeit aus dem ersten Hohlzylinder in den Innenraum der Kartusche gedrückt wird, als auch die Luft aus dem Innenraum der Kartusche gezogen wird.

Dies stellt sicher, dass die Vorrichtung auf eine einfache Art und Weise und ohne zusätzliche Bauteile oder eine elektronische Regelung oder Steuerung aufgebaut werden kann. Die Vorrichtung wird dadurch auch an Orten ohne Energieversorgung einsetzbar.

Des Weiteren kann auch vorgesehen sein, dass der erste Hohlzylinder zwischen dem Monomer-Behälter oder dem Anschluss für den Monomer-Behälter und dem Innenraum der Kartusche angeordnet ist. Dabei kann bevorzugt vorgesehen sein, dass in der Verbindungsleitung zwischen dem Monomer-Behälter oder dem Anschluss für den Monomer-Behälter und dem Innenraum der Kartusche angeordnet ist.

Hierdurch kann auf eine zusätzliche Leitung verzichtet werden und es ist auch kein Umstecken der Verbindungsleitung notwendig. Dies vereinfacht die Anwendung der Vorrichtung zusätzlich.

Dass der erste Hohlzylinder zwischen dem Monomer-Behälter für die Monomerflüssigkeit oder dem Anschluss für den Monomer-Behälter und der Kartusche angeordnet ist, bedeutet nicht, dass der erste Hohlzylinder geometrisch dazwischen angeordnet ist, sondern bezüglich der Fluidverbindungen also der Flussrichtung der Monomerflüssigkeit, wenn diese aus dem geöffneten Monomer-Behälter in Richtung der Kartusche fließt, beziehungsweise gepumpt wird, zwischen dem Monomer-Behälter oder dem Anschluss für den Monomer-Behälter und der Kartusche angeordnet ist.

Mit einer Weiterbildung der vorliegenden Erfindung wird vorgeschlagen, dass der Pumpkolben und der erste Hohlzylinder eine geringere Querschnittsfläche aufweisen als der Vakuumkolben und der zweite Hohlzylinder, vorzugsweise der Vakuumkolben und der zweite Hohlzylinder eine mindestens doppelt so große Querschnittsfläche aufweisen wie der Pumpkolben und der erste Hohlzylinder.

Damit wird erreicht, dass mit dem Vakuumkolben eine größere Menge Gas aus dem Innenraum der Kartusche evakuiert werden kann. Gleichzeitig ist der Kraftaufwand zum Bedienen des Pumpkolbens nicht zu hoch, so dass eine manuelle Bedienung der Vorrichtung, insbesondere der miteinander verbundenen Pump- und Vakuumkolben leicht möglich ist.

Es kann auch vorgesehen kann, dass der Pumpkolben und der Vakuumkolben miteinander verbunden sind, bevorzugt fest miteinander verbunden sind, besonders bevorzugt einteilig aufgebaut sind.

Hierdurch wird ein stabiler und einfacher Aufbau erreicht.

Dabei kann auch vorgesehen sein, dass die der der Vakuumleitung zugewandte Seite des Vakuumkolbens auf der Rückseite des Pumpkolbens angeordnet ist und dementsprechend die Verbindungsleitung zugewandte Seite des Pumpkolbens auf der Rückseite des Vakuumkolbens angeordnet ist.

Der Pumpkolben und der Vakuumkolben können erfindungsgemäß also durch einen gemeinsamen Pump-Vakuum-Kolben realisiert sein, wobei dadurch der Aufbau der Vorrichtung vereinfacht wird. Hierdurch wird ferner ein besonders einfach anzuwendendes Zweiseitenkolbenprinzip realisiert, mit dem beide Seiten des kombinierten Pump-Vakuum-Kolbens, beziehungsweise beide Stirnflächen des kombinierten Pump-Vakuum-Kolbens, zum Leisten der Arbeit aufgrund einer gemeinsamen Bewegung verwendet werden, nämlich sowohl das Pumpen der Monomerflüssigkeit in den Innenraum der Kartusche als auch das Erzeugen eines Unterdrucks zum Evakuieren des Innenraums der Kartusche.

Es wird ferner vorgeschlagen, dass die Vakuumleitung durch den Austragskolben mit dem Innenraum der Kartusche verbunden ist, wobei vorzugsweise im Austragskolben ein Filter oder ein Sieb angeordnet ist über den oder das die Vakuumleitung mit dem Innenraum der Kartusche verbunden ist.

Vorzugsweise ist der Austragskolben zweiteilig mit einem Sterilisationskolben und einem Förderkolben aufgebaut, wobei der Sterilisationskolben und der Förderkolben miteinander verbindbar sind.

Dadurch kann der Anschluss für die Vakuumleitung vorher zum Evakuieren und zum Sterilisieren mit einem Gas verwendet werden. Zudem ist der Anschluss so gegenüberliegend zu einer Austragsöffnung der Kartusche angeordnet, durch die auch die Monomerflüssigkeit in den Innenraum der Kartusche gedrückt wird, in die also die Verbindungsleitung in den Innenraum der Kartusche mündet. Der Unterdruck kann so die Monomerflüssigkeit in die Kartusche einsaugen und damit die Befüllung der Kartusche mit der Monomerflüssigkeit zusätzlich unterstützen.

Bei einer Weiterbildung der vorliegenden Erfindung kann vorgesehen sein, dass der Monomer-Behälter in einem flexiblen Ampullen-Behälter angeordnet ist oder anzuordnen ist, wobei vorzugsweise in dem Ampullen-Behälter zumindest eine Belüftungsöffnung vorgesehen ist.

Hierdurch kann eine Ampulle als Monomer-Behälter auf einfache Weise geöffnet werden, indem der Ampullen-Behälter gebogen wird und dadurch die Ampulle aufgebrochen wird. Die Belüftungsöffnung hilft dabei, dass die Monomerflüssigkeit leicht auslaufen kann. Zudem kann die zumindest eine Belüftungsöffnung zur Sterilisation der Vorrichtung mit Ethylenoxid verwendet werden.

Gemäß einer bevorzugten Weiterentwicklung der vorliegenden Erfindung kann vorgesehen sein, dass in der Kartusche eine Mischeinrichtung angeordnet ist, die von außen bedienbar ist, wobei vorzugsweise die Mischeinrichtung über eine Mischstange bedienbar ist, die durch eine Durchführung in dem Austragskolben ins Innere der Kartusche geführt und beweglich gelagert ist.

Besonders bevorzugt ist die Mischstange in der Durchführung drehbar und in Längsrichtung verschiebbar gelagert. Mit der Mischeinrichtung kann der Inhalt des Innenraums der Kartusche bequem mit der Mischstange durchmischt werden. Bei der Verwendung von niedrig-viskosen Knochenzementen kann auf die Verwendung einer Mischstange und einer Mischeinrichtung verzichtet werden, weil die Monomerflüssigkeit vor dem Anquellen des Zementpulvers die Luft der Porenräume zwischen den Zementpulverpartikeln verdrängt hat und die Zementpulverpartikel benetzt hat.

Es kann auch vorgesehen sein, dass der Austragskolben für Pulver undurchlässig ist, wobei bevorzugt in dem Austragskolben ein Porenfilter angeordnet ist, der für Gas durchlässig ist und für Pulver undurchlässig ist.

Der Porenfilter kann bevorzugt als Porenscheibe ausgebildet sein. Durch die Undurchlässigkeit für Pulver kann verhindert werden, dass das Zementpulver aus dem Inneren der Kartusche austreten kann. Wenn der Austragskolben gasdurchlässig ist, kann der Innenraum durch den Austragskolben evakuiert und mit einem Gas, wie beispielsweise Ethylenoxid, sterilisiert werden. Die Vakuumleitung mündet bevorzugt durch den Austragskolben, insbesondere durch die Porenscheibe, in den Innenraum der Kartusche.

Bevorzugt kann auch vorgesehen sein, dass das Zementpulver im Innenraum der Kartusche enthalten ist.

Es kann auch vorgesehen sein, dass die Monomerflüssigkeit in dem Monomer-Behälter enthalten ist. Hierdurch bildet die Vorrichtung ein fertiges Full-Prepacked-Mischsystem, das nicht vor der Anwendung mit dem Zementpulver gefüllt werden muss. In der Kartusche wird das Zementpulver vor der Verwendung separat zur Monomerflüssigkeit gelagert.

Es wird auch vorgeschlagen, dass zwischen der Verbindungsleitung und dem Innenraum der Kartusche ein für das Zementpulver undurchlässiger und für die Monomerflüssigkeit durchlässiger Filter angeordnet ist.

Hierdurch kann verhindert werden, dass das Zementpulver in die Verbindungsleitung eindringt und dort beim Einleiten der Monomerflüssigkeit polymerisiert und so ungewollt die Verbindungsleitung verstopft beziehungsweise verklebt.

Bevorzugt Ausführungsformen können vorsehen, dass die Vorrichtung einen Standfuß aufweist, in dem zumindest ein Teil der Verbindungsleitung angeordnet ist, wobei die Kartusche lösbar mit dem Standfuß verbunden ist, insbesondere über ein Schraubgewinde lösbar mit dem Standfuß verbunden ist, wobei bevorzugt gegebenenfalls der für das Zementpulver undurchlässige und für die Monomerflüssigkeit durchlässige Filter in dem Standfuß der Vorrichtung angeordnet ist, besonders bevorzugt in der Verbindung zur Kartusche des Standfußes angeordnet ist.

Hierdurch kann die Vorrichtung leicht aufgestellt und einfach bedient werden.

Dabei kann vorgesehen sein, dass der erste Hohlzylinder, der zweite Hohlzylinder und der Monomer-Behälter oder der erste Hohlzylinder, der zweite Hohlzylinder und der Anschluss zur Befestigung des Monomer-Behälters mit dem Standfuß verbunden sind, bevorzugt unlösbar mit dem Standfuß verbunden sind.

Hierdurch wird ein besonders einfacher und kostengünstiger Aufbau der Vorrichtung ermöglicht.

Es wird mit der vorliegenden Erfindung auch vorgeschlagen, dass der Monomer-Behälter für die Monomerflüssigkeit oder der Anschluss zur Befestigung des Monomer-Behälters an einer Mantelfläche des ersten Hohlzylinders in den ersten Hohlzylinder mündet, bevorzugt unmittelbar unterhalb des Pumpkolbens in den ersten Hohlzylinder mündet.

Hierdurch kann sichergestellt werden, dass die Monomerflüssigkeit vollständig in den Hohlzylinder fließen kann und den Hohlzylinder füllen kann. Zudem kann so Luft an dieser Stelle besonders leicht aus dem Hohlzylinder austreten.

Des Weiteren kann vorgesehen sein, dass die Vorrichtung ein Öffnungsmittel zum Öffnen des Monomer-Behälters aufweist, mit dem der Monomer-Behälter innerhalb der Vorrichtung zu öffnen ist, wobei bevorzugt in der Verbindung zum ersten Hohlzylinder ein Sieb oder ein Filter angeordnet ist, mit dem Bruchstücke oder Fetzen des geöffneten Monomer-Behälters zurückgehalten werden können.

Bevorzugt kann dabei vorgesehen sein, dass der Monomer-Behälter eine aufbrechbare Glasampulle ist.

Durch das Öffnungsmittel als Teil der Vorrichtung kann die Vorrichtung auch langfristig zum Lagern des Monomers verwendet werden. Eine geeignetes Öffnungsmittel ist beispielsweise aus dem Patent DE 10 2010 026 496 B4 bekannt.

Des Weiteren kann vorgesehen sein, dass der Monomer-Behälter oberhalb der der Verbindung zum ersten Hohlzylinder angeordnet ist.

Dadurch kann die Monomerflüssigkeit aus dem Monomer-Behälter nach dem Öffnen des Monomer-Behälters aufgrund der Gravitation in den ersten Hohlzylinder fließen. Alternativ könnte der Monomer-Behälter auch ausgepresst werden und so Monomer in den ersten Hohlzylinder fließen.

Bevorzugte Vorrichtungen können auch dadurch auszeichnen, dass die Verbindungsleitung an der unteren Seite mit dem ersten Hohlzylinder verbunden ist, bevorzugt am tiefsten Punkt des ersten Hohlzylinders mit dem ersten Hohlzylinder verbunden ist, wobei besonders bevorzugt der Pumpkolben auf der gegenüberliegenden Seite des ersten Hohlzylinders angeordnet ist.

Hierdurch kann die Monomerflüssigkeit mit dem Pumpkolben vollständig aus dem ersten Hohlzylinder abfließen beziehungsweise herausgedrückt werden.

Es wird ferner vorgeschlagen, dass der ersten Hohlzylinder auf der dem Pumpkolben gegenüberliegenden Seite einen kegelförmigen, halbkugelförmigen oder anderen sich nach unten verjüngenden Boden aufweist, wobei bevorzugt die dem Boden des ersten Hohlzylinders zugewandte Fläche des Pumpkolbens eine Negativform des Bodens bildet.

Hierdurch kann die Monomerflüssigkeit mit dem Pumpkolben vollständig aus dem ersten Hohlzylinder abfließen beziehungsweise herausgedrückt werden. Das bedeutet, dass die gesamte Monomerflüssigkeit zum tiefsten Punkt des ersten Hohlzylinders fließt und keine "toten" Bereiche vorhanden sind, in denen Monomerflüssigkeit bei Betätigung des Pumpkolbens zurück bleibt. Durch die Anpassung der Form des Pumpkolbens an die innere Form des ersten Hohlzylinders wird die gesamte Monomerflüssigkeit aus dem ersten Hohlzylinder durch den Pumpkolben bei Bewegung des Pumpkolbens in Richtung der Öffnung zur Verbindungsleitung gepresst, ohne dass Rückstände der Monomerflüssigkeit im ersten Hohlzylinder verbleiben. Weiterhin wird durch diese kegelförmige oder kugelförmige beziehungsweise passende Gestalt der Stirnseite des Pumpkolbens gewährleistet, dass bei Bewegung des Pumpkolbens nach unten beziehungsweise in Richtung des Standfußes, die Luft über der Monomerflüssigkeit im ersten Hohlzylinder durch die Öffnung in der Mantelfläche des ersten Hohlzylinders entweichen kann und keine Luftblasen oberhalb der Monomerflüssigkeit beim Transferieren der Monomerflüssigkeit in den Innenraum der Kartusche beziehungsweise in das Zementpulver verbleiben.

Bevorzugte erfindungsgemäße Vorrichtungen können sich auch dadurch auszeichnen, dass der Pumpkolben manuell axial im ersten Hohlzylinder bewegt werden kann, bevorzugt manuell axial in den ersten Hohlzylinder eingepresst werden kann, und/oder der Vakuumkolben manuell axial im zweiten Hohlzylinder bewegt werden kann.

Damit ist es möglich, die Monomer-Flüssigkeit manuell aus dem ersten Hohlzylinder auszupressen und in den Innenraum der Kartusche zu überführen, beziehungsweise das Vakuum manuell im zweiten Hohlzylinder zu erzeugen, um Luft aus der Kartusche zu evakuieren.

Zur Vereinfachung der Bedienung und für eine höhere Variabilität erfindungsgemäßer Vorrichtungen kann auch vorgesehen sein, dass der erste Hohlzylinder transparent ist und Markierungen zur Füllstandsmenge einer Flüssigkeit in dem ersten Hohlzylinder aufweist.

Dadurch kann eine durch die Markierungen bestimmte Menge der Monomerflüssigkeit in den ersten Hohlzylinder gefüllt werden beziehungsweise aus dem ersten Hohlzylinder in den Innenraum der Kartusche eingepresst werden. So ergibt sich die Möglichkeit mit der Vorrichtung einen Knochenzementteig mit einer durch die Menge der Monomerflüssigkeit vorgegebenen Konsistenz herzustellen. Alternativ kann der ersten Hohlzylinder auch nicht transparent sein und Markierungen an einem aus dem Gehäuse der Vorrichtung ragenden Bedienelement des Pumpkolbens beziehungsweise Vakuumkolbens vorgesehen sein, um einen definierten Vortrieb des Pumpkolbens zu ermöglichen und damit ein definiertes Volumen der Monomerflüssigkeit aus dem ersten Hohlzylinder pressen zu können. Mit derartigen Aufbauten ist es also möglich, sowohl das gesamte Volumen der Monomerflüssigkeit aus dem ersten Hohlzylinder in das Zementpulver im Innenraum der Kartusche zu pressen als auch nur bestimmte Teilvolumen der Monomerflüssigkeit aus dem ersten Hohlzylinder in das Zementpulver zu transferieren. Dadurch kann das Verhältnis von Monomerflüssigkeit zur Pulvermenge eingestellt werden, wodurch das zeitliche Erreichen der Klebfreiheit des gebildeten Zementteigs und die Viskosität des Knochenzements gezielt gesteuert werden kann.

Es kann auch vorgesehen sein, dass der erste Hohlzylinder und/oder der zweite Hohlzylinder ein Innengewinde aufweist und der Pumpkolben und/oder der Vakuumkolben ein dazu passendes Außengewinde aufweist, so dass der Pumpkolben und/oder der Vakuumkolben in den ersten Hohlzylinder und/oder zweiten Hohlzylinder einschraubbar ist, um die Monomerflüssigkeit aus dem ersten Hohlzylinder in den Innenraum der Kartusche zu pressen und/oder um Luft aus dem Innenraum der Kartusche in den zweiten Hohlzylinder zu ziehen.

Auch hierdurch kann eine definierte Menge der Monomerflüssigkeit aus dem ersten Hohlzylinder in den Innenraum der Kartusche eingepresst werden. Dadurch ergibt sich die Möglichkeit mit der Vorrichtung einen Knochenzementteig mit einer durch die Menge der Monomerflüssigkeit bestimmten Konsistenz herzustellen. Zudem kann so der Unterdruck in dem zweiten Hohlzylinder mit einer großen Kraft erzeugt werden. Gleichzeitig ist die Gefahr, dass der Vakuumkolben sich aufgrund des Unterdrucks wieder ungewollt in Richtung des Anschlusses zur Vakuumleitung bewegt durch das Gewinde reduziert. Wenn der Vakuumkolben einen größeren Querschnitt hat als Pumpkolben, was erfindungsgemäß bevorzugt ist, dann kann es ausreichen, dass der Vakuumkolben ein Außengewinde aufweist, das in ein Innengewinde des zweiten Hohlzylinders zu schrauben ist. Dies gilt insbesondere dann, wenn der Pumpkolben und der Vakuumkolben als gemeinsamer Pump-Vakuum-Kolben ausgeführt sind.

Ferner kann vorgesehen sein, dass die Vorrichtung zumindest eine gespannte Druckfeder und zumindest eine Arretierung aufweist, wobei die Druckfeder, der Vakuumkolben und/oder der Pumpkolben mit der zumindest einen Arretierung lösbar arretiert ist oder sind, wobei bei gelöster Arretierung die zumindest eine Druckfeder einen Druck auf den Pumpkolben und/oder den Vakuumkolben ausübt, so dass der Pumpkolben in den ersten Hohlzylinder gepresst wird und/oder der Vakuumkolben von dem Anschluss zur Vakuumleitung weg in den zweiten Hohlzylinder gedrückt wird.

Für die Ausführung, bei der der erste und/oder zweite Hohlzylinder ein Innengewinde und der Pumpkolben und/oder Vakuumkolben ein Außengewinde aufweist, kann analog hierzu zumindest eine gespannte Schneckenfeder vorgesehen sein, die nach Lösen zumindest einer Arretierung den Pumpkolben in den ersten Hohlzylinder schraubt und/oder Vakuumkolben im zweiten Hohlzylinder schraubt.

Diese Maßnahmen haben den Vorteil, dass die Bedienung der Vorrichtung vereinfacht wird. Zudem können so mögliche Fehlbedienungen verhindert werden.

Ferner kann vorgesehen sein, dass der Austragskolben mit einer lösbaren Rastvorrichtung mit der Kartusche verbunden ist, wobei die Rastvorrichtung manuell, insbesondere durch axiale Krafteinwirkung lösbar ist, so dass der Austragskolben axial im Innenraum der Kartusche bewegt werden kann.

Hierdurch kann eine ungewollte Bewegung des Austragskolbens, wie sie beispielsweise durch ein Vakuum oder ein Unterdruck im Innenraum der Kartusche verursacht werden kann, verhindert werden.

Es wird mit der Erfindung auch vorgeschlagen, dass die Verbindungsleitung zwischen dem ersten Hohlzylinder und dem Innenraum der Kartusche eine nach oben weisende Schlaufe aufweist, wobei der höchste Punkt der Schlaufe oberhalb einer Einmündung des Monomer-Behälters oder des Anschlusses für den Monomer-Behälter in den ersten Hohlzylinder liegt.

Damit kann verhindert werden, dass die Monomerflüssigkeit beim Einfüllen in den ersten Hohlzylinder bereits durch die Verbindungsleitung in den Innenraum der Kartusche gelangt. Diese umgekehrt U-förmige Schlaufe der Verbindungsleitung erreicht, dass vor Bewegung des Pumpkolbens in Richtung der Verbindungsleitung zur Kartusche die Monomerflüssigkeit im ersten Hohlzylinder bis zur Höhe des Scheitelpunkts in der Verbindungsleitung verbleibt, wodurch ein vorzeitiger Eintritt der Monomerflüssigkeit zum Zementpulver verhindert wird. Insbesondere bei hochviskosen Zementen kann ein vorzeitiger Kontakt von schon geringen Volumina der Monomerflüssigkeit mit dem Zementpulver zur Verklebung der Verbindungsleitung oder eines als Düse ausgebildeten Leitungsmittels, wie in US 8 662 736 B2 beschrieben, führen. Die Verbindungsleitung kann transparent oder transluzent sein, damit der Anwender den Monomertransfer visuell kontrollieren kann. Hierzu kann insbesondere ein Sichtfenster in der Vorrichtung vorgesehen sein, durch das die Schlaufe mit dem höchsten Scheitelpunkt zu erkennen ist.

Des Weiteren kann erfindungsgemäß vorgesehen sein, dass das Volumen im ersten Hohlzylinder kleiner oder gleich dem Volumen der Monomerflüssigkeit in dem Monomer-Behälter ist.

Dadurch wird verhindert, dass bei Betätigung des Pumpkolbens Luft mit in das Zementpulver gepresst werden kann.

Ferner kann vorgesehen sein, dass der Innenraum der Kartusche an der unteren Seite mit der Verbindungsleitung flüssigkeitsdurchlässig verbunden ist.

Die Verbindungsleitung kann in der Stirnseite des Innenraums in eine Düse gemäß US 8 662 736 B4 münden. Diese Düse verhindert einen Zutritt von Zementpulver in das Leitungsmittel.

Die der vorliegenden Erfindung zugrundeliegenden Aufgaben werden auch gelöst durch ein Verfahren zum Mischen eines Knochenzements, insbesondere mit einer erfindungsgemäßen Vorrichtung, mit den chronologischen Schritten
A) ein Monomer-Behälter wird geöffnet,
B) eine Monomerflüssigkeit fließt aus dem Monomer-Behälter in einen ersten Hohlzylinder, wobei der erste Hohlzylinder auf einer Seite durch einen Pumpkolben begrenzt ist,
C) der Pumpkolben wird in den ersten Hohlzylinder eingedrückt und die Monomerflüssigkeit dadurch aus dem ersten Hohlzylinder und durch eine Verbindungsleitung in den Innenraum einer Kartusche eingepresst, wobei sich in dem Innenraum der Kartusche ein Zementpulver befindet,
D) mit der Bewegung des Pumpkolbens wird ein mit dem Pumpkolben verbundener oder parallel angetriebener Vakuumkolben in einem zweiten Hohlzylinder von einem Anschluss einer Vakuumleitung weg bewegt, wobei durch die Bewegung des Vakuumkolbens der Gasdruck zwischen dem Anschluss der Vakuumleitung und dem Vakuumkolben im zweiten Hohlzylinder verringert wird und Gas durch die Vakuumleitung aus dem an die Vakuumleitung angeschlossenen Innenraum der Kartusche evakuiert wird, und
E) die Monomerflüssigkeit und das Zementpulver werden in dem Innenraum der Kartusche gemischt.

Bevorzugt fließt die Monomerflüssigkeit durch Einwirkung der Schwerkraft in den Hohlzylinder.

Dabei kann vorgesehen sein, dass die Monomerflüssigkeit und das Zementpulver erst dann in dem Innenraum der Kartusche gemischt werden, wenn der Pumpkolben vollständig oder bis zu einer Markierung in den ersten Hohlzylinder eingedrückt wurde, wobei die Markierung ein Maß für die in den Innenraum der Kartusche eingeleitete Monomerflüssigkeit ist.

Hierdurch kann sichergestellt werden, dass der erzeugte Knochenzementteig die gewünschte Konsistenz durch die gewünschte Beimengung von Monomerflüssigkeit erhält.

Bei erfindungsgemäßen Verfahren kann auch vorgesehen sein, dass die Luft aus dem Innenraum der Kartusche auf der der Einmündung der Verbindungsleitung gegenüberliegenden Seite durch die Vakuumleitung abgesaugt wird, wobei vorzugsweise die Verbindungsleitung an der Unterseite des Innenraums in den Innenraum der Kartusche mündet und die Vakuumleitung an der Oberseite des Innenraums in den Innenraum der Kartusche mündet.

Hierdurch kann sichergestellt werden, dass gleichzeitig die Monomerflüssigkeit in den Innenraum der Kartusche geleitet und Luft aus dem Innenraum der Kartusche evakuiert werden kann. Zudem kann so vermieden werden, dass die Monomerflüssigkeit ungewollt in die Vakuumleitung eindringt.

Es wird des Weiteren vorgeschlagen, dass die Monomerflüssigkeit und das Zementpulver in dem Innenraum mit einer Mischeinrichtung gemischt werden, indem die Mischeinrichtung durch Bewegen einer drehbar und in Längsrichtung verschiebbar in den Innenraum der Kartusche geführten Mischstange bedient wird, wobei bevorzugt nach dem Mischen die Mischstange bis zum Anschlag aus dem Innenraum der Kartusche herausgezogen wird und besonders bevorzugt die Mischstange nach dem Herausziehen bis zum Anschlag an einer Sollbruchstelle abgebrochen wird.

Hierdurch kann das Verfahren einfach durch manuelle Bedienung durchgeführt werden.

Ferner kann vorgesehen sein, dass der Monomer-Behälter geöffnet wird, in dem ein Öffnungsmittel bedient oder ausgelöst wird, wobei bevorzugt der Monomer-Behälter durch das Öffnungsmittel aufgebrochen wird.

Hierdurch kann der Monomer-Behälter in der Vorrichtung geöffnet werden, so dass die gesamte Vorrichtung nach außen abgeschlossen ist.

Ferner kann vorgesehen sein, dass der Pumpkolben mit einem gespannten elastischen Federelement in den ersten Hohlzylinder eingedrückt wird und/oder der Vakuumkolben mit einem gespannten elastischen Federelement im zweiten Hohlzylinder bewegt wird, wobei hierfür vorzugsweise zuvor zumindest eine Arretierung gelöst wird, die in den Pumpkolben, den Vakuumkolben und/oder das Federelement greift oder greifen.

Damit wird eine weitere Automatisierung des erfindungsgemäßen Verfahrens erreicht und zudem mögliche Fehlbedienungen verhindert.

Erfindungsgemäß kann auch vorgesehen sein, dass die Kartusche mit dem fertig gemischten Zementteig von der Verbindungsleitung, der Vakuumleitung, dem ersten Hohlzylinder, dem zweiten Hohlzylinder und dem Monomer-Behälter gelöst wird und der fertig gemischte Zementteig durch Vortreiben eines Austragskolbens, der axial beweglich in der Kartusche gelagert ist und der den Innenraum der Kartusche auf einer Seite begrenzt, aus dem Innenraum der Kartusche ausgetragen wird.

Der Erfindung liegt die überraschende Erkenntnis zugrunde, dass eine Monomerflüssigkeit mit Hilfe eines Pumpkolbens von unten in den Innenraum einer Kartusche eingepresst werden kann und dass durch eine gleichzeitige Bewegung eines Vakuumkolbens in einem Hohlzylinder, im Innenraum der Kartusche ein Unterdruckerzeugt werden kann, so dass sich hierbei keine störenden Lufteinschlüsse im Knochenzement bilden. Gleichzeitig kann mit der Vorrichtung weitgehend auf externe Energiequellen oder interne Energiespeicher verzichtet werden, da der Pumpkolben und der Vakuumkolben manuell betrieben werden können. Insbesondere müssen keine Vakuumquellen und langfristig vakuumdichten Anschlüsse und Bauteile verwendet werden, was den Einsatz an weniger entwickelten Orten sowie im Einsatz vor Ort oder in Feldlazaretten erheblich erleichtert. Zudem sind erfindungsgemäße Full-Prepacked-Mischsysteme unanfälliger für mögliche Störungen und dadurch mit sehr hoher Wahrscheinlichkeit einsatzbereit, weil keine Vakuumlecks auftreten können.

Beispielsweise kann bei einer erfindungsgemäßen Vorrichtung beziehungsweise einem erfindungsgemäßen Verfahren vorgesehen sein, dass nach Öffnung eines Monomer-Behälters die Monomerflüssigkeit durch Schwerkrafteinwirkung in einen Hohlzylinder fließt, aus diesem durch manuelle Betätigung des Pumpkolbens in den Innenraum einer Kartusche gepresst wird, die Zementpulver enthält. Das bedeutet, dass im Gegensatz zu den bisher marktüblichen Mischsystemen der Transfer der Monomerflüssigkeit nicht durch Vakuum sondern durch Druckeinwirkung erfolgt. Ein solcher manuell zu betätigender Monomertransfer durch Druckeinwirkung kann kostengünstig mit einfachen, durch Kunststoffspritzgießen herzustellenden Kunststoffteilen realisiert werden. Gleichzeitig wird zur Vermeidung oder Reduzierung von Lufteinschlüssen im Zementteig durch die gleichzeitige Bewegung des Vakuumkolbens ein Unterdruck in dem Innenraum der Kartusche erzeugt. Der besondere Vorteil der erfindungsgemäßen Vorrichtung besteht darin, dass die Vorrichtung ohne äußere Hilfsmittel, wie durch Druckluft angetriebene Vakuumpumpen, und ohne externe Energiequellen, wie Druckluft oder Strom, betrieben werden kann. Die erfindungsgemäße Vorrichtung ist dadurch autonom verwendbar und kann selbst unter schwierigsten Operationsbedingungen verwendet werden. Mit der erfindungsgemäßen Vorrichtung wird ein geschlossenes Full-Prepacked-Mischsystem für preissensitive Märkte zur Verfügung gestellt.

Es wurde im Rahmen der vorliegenden Erfindung gefunden, dass bei der durch Druck verursachten Einleitung der Monomerflüssigkeit in das Zementpulver von der Unterseite des Innenraums der Kartusche die Monomerflüssigkeit in Form einer einheitlichen Front von unten nach oben wandert. Dadurch wird die Luft, die sich in den Zwischenräumen zwischen den Zementpulverpartikeln befindet, verdrängt und nach oben heraus gedrückt. Gleichzeitig wird parallel die Luft aktiv durch die Bewegung des Vakuumkolbens herausgezogen. Lufteinschlüsse werden dadurch vermieden oder zumindest reduziert. Es wurde im Rahmen der vorliegenden Erfindung gefunden, dass ein mit einer erfindungsgemäßen Vorrichtung und einem erfindungsgemäßen Verfahren hergestellter Knochenzementteig sehr weitgehend frei von Lufteinschlüssen ist und in der Qualität einem unter Vakuum gemischten Zementteig entspricht.

Eine beispielhafte erfindungsgemäße Vorrichtung zur Lagerung und Vermischung von Polymethylmethacrylat-Knochenzement aufweisend
a) eine Kartusche, wobei eine erste Stirnseite des Innenraums der Kartusche mit mindestens einem für Pulver undurchlässigen Austragskolben verschlossen ist und wobei die zweite Stirnseite des Innenraums der Kartusche für Pulver undurchlässig ist, wodurch durch den Austragskolben und die zweite Stirnseite begrenzte Innenraum der Kartusche gebildet wird, in dem Zementpulver angeordnet ist,
b) wobei der Innenraum der Kartusche über eine für Pulver undurchlässige, flüssigkeitsdurchlässige Öffnung mit einer flüssigkeitsdurchlässigen Verbindungsleitung (Leitungsmittel) mit einem zylinderförmigen ersten Hohlraum in einem ersten Hohlzylinder verbunden ist,
c) wobei an der Mantelfläche des ersten Holzylinders eine für Flüssigkeiten durchlässige Öffnung angeordnet ist, welche den ersten Hohlraum, mit einem Öffnungsmittel für einen Monomerflüssigkeitsbehälter verbindet, das mit einem Monomerflüssigkeitsbehälter verbunden ist, der oberhalb der Öffnung in der Mantelfläche des ersten Hohlzylinders angeordnet ist,
d) einem im zylinderförmigen ersten Hohlraum angeordneten Pumpkolben, beziehungsweise ein kombinierter Pump-Vakuum-Kolben, der axial im ersten Hohlzylinder verschiebbar ist,
e) wobei der Austragskolben aus einem Dichtkolben und einem Sterilisationskolben zusammengesetzt ist, wobei der Dichtkolben über dem Sterilisationskolben angeordnet ist, der Dichtkolben nach Einschieben in den Innenraum der Kartusche, diesen Innenraum gasdicht verschließt und der eine gasdurchlässige Durchführung besitzt, die den Raum unterhalb des Dichtkolbens mit dem Raum oberhalb des Dichtkolbens verbindet wobei die Durchführung an der Oberseite als Vakuumanschluss ausgebildet ist,
f) wobei der erste Hohlzylinder an seiner dem Fußteil entgegengesetzten Stirnseite gasdicht verschlossen ist,
g) wobei ein zweiter Hohlraum durch die Oberseite des Pumpkolbens und eine Verlängerung des an der Stirnseite gasdicht verschlossenen ersten Hohlzylinder beziehungsweise eines zweiten Hohlzylinders gebildet ist, so dass die Oberseite des Pumpkolbens einen Vakuumkolben bildet und hierdurch ein kombinierter Pump-Vakuum-Kolben gebildet ist,
h) wobei in der gasdicht verschlossenen Stirnseite des zweiten Hohlzylinders eine gasdurchlässige Durchführung ausgebildet ist, die mit der gasdurchlässigen Durchführung des Austragskolbens beziehungsweise des Dichtkolbens mit einem gasdurchlässigen Verbindungsmittel verbunden ist.

Dabei kann der Pumpkolben und damit der Vakuumkolben durch ein gasdicht geführtes Antriebselement axial im ersten beziehungsweise zweiten Hohlzylinder bewegt werden.

Die Idee der Erfindung beruht darauf, dass nach Öffnung eines Monomerbehälters mit einer Öffnungsvorrichtung, zum Beispiel mit einer Vorrichtung nach DE 10 2010 026 496 B4, die Monomerflüssigkeit durch Schwerkrafteinwirkung in den ersten Hohlzylinder fließt, aus diesem durch manuelle Betätigung des Pumpkolbens in die Kartusche gepresst wird, die Zementpulver enthält. Der erste Hohlzylinder beziehungsweise der zweite Hohlzylinder ist an seiner dem Fußteil abgewandten Stirnseite gasdicht verschlossen. Bei Bewegung des Vakuumkolbens in Richtung des Fußteils, wobei dabei durch die Bewegung des Pumpkolbens bei einem kombinierten Pump-Vakuum-Kolben die Monomerflüssigkeit in den Innenraum der Kartusche gepresst wird, entsteht gleichzeitig ein Unterdruck im Hohlraum oberhalb des Vakuumkolbens im zweiten Hohlzylinder. Das bedeutet, es entsteht gleichzeitig ein Unterdruck, der über eine Durchführung und über ein gasdurchlässiges Verbindungsmittel (die Vakuumleitung) in den Innenraum der Kartusche geleitet wird. Wenn bei der Bewegung des Pump-Vakuum-Kolbens das Volumen im Hohlraum zwischen dem Vakuumkolben und dem Anschluss zur Vakuumleitung größer ist als das Volumen der auszupressenden Monomerflüssigkeit im ersten Hohlzylinder, dann entsteht in der ersten Kartusche ein Unterdruck. Dieser Effekt kann erreicht werden, in dem der kombinierte Pump-Vakuum-Kolben aus zwei zylinderförmigen Kolben, nämlich den Pumpkolben und dem Vakuumkolben gebildet ist, wobei der untere Zylinder (Pumpkolben) einen kleineren Außendurchmesser als der ober Zylinder (Vakuumkolben) hat. Das bedeutet, dass der kombinierte Pump-Vakuum-Kolben doppelt-wirkend ist. Eine Seite des kombinierten Pump-Vakuum-Kolbens hat bei axialer Bewegung des Pump-Vakuum-Kolbens eine Pumpfunktion zur Erzeugung von Überdruck und die andere Seite des Pump-Vakuum-Kolbens erzeugt gleichzeitig einen Unterdruck.

Ein weiterer Vorteil erfindungsgemäßer Vorrichtung und Verfahren besteht darin, dass ein Monomertransfer durch die Auspressbewegung des Pumpkolbens immer gewährleistet ist, unabhängig, ob der Anwender den Innenraum der Kartusche gasdicht mit dem Pumpkolben beziehungsweise dem Vakuumkolben verschlossen hat oder nicht. Dadurch ist die Handhabungssicherheit deutlich gegenüber bisher bekannten Full-Prepacked-Mischsystemen erhöht, bei denen der Transfer der Monomerflüssigkeit nur durch das angelegte Vakuum bewirkt wird, das je nach verwendeter Energiequelle mehr oder wenig stabil vorhanden ist.

Erfindungsgemäß ist beispielsweise ein Verfahren mit der oben genannten beispielhaften Vorrichtung, das durch folgende nacheinander ablaufende Schritte charakterisiert ist,
a) durch Betätigung des Öffnungsmittels wird der Monomerflüssigkeitsbehälter geöffnet,
b) dass die Monomerflüssigkeit durch Einwirkung der Schwerkraft durch die Öffnung in der Mantelfläche des ersten Hohlzylinders in den Hohlraum des ersten Hohlzylinders fließt,
c) dass nach vollständigen Transfer der Monomerflüssigkeit in den Hohlraum des ersten Hohlzylinders der Pumpkolben beziehungsweise der kombinierte Pump-Vakuum-Kolben manuell in Richtung des Fußteils der Vorrichtung gedrückt wird,
d) wodurch die Monomerflüssigkeit durch die Verbindungsleitung (das Leitungsmittel) in den Innenraum der Kartusche in das Zementpulver gedrückt wird,
e) dass nach vollständigen Transfer der Monomerflüssigkeit vom ersten Hohlraum in den Innenraum der Kartusche das Gemisch aus Zementpulver und Monomerflüssigkeit manuell durch Betätigung einer Mischstange gemischt wird,
f) dass dann die Mischstange nach oben gezogen wird,
g) die Mischstange an einer Sollbruchstelle abgebrochen wird und
h) dass dann die Kartusche vom Fußteil getrennt wird.

Ein weiteres beispielhaftes Verfahren mit der oben genannten beispielhaften Vorrichtung ist durch folgende nacheinander ablaufende Schritte charakterisiert,
a) durch Betätigung des Öffnungsmittels wird der Monomerflüssigkeitsbehälter geöffnet,
b) dass die Monomerflüssigkeit durch Einwirkung der Schwerkraft durch die Öffnung in der Mantelfläche des ersten Hohlzylinders in den Hohlraum des ersten Hohlzylinders fließt,
c) dass nach vollständigen Transfer der Monomerflüssigkeit in den ersten Hohlraum eine Arretierung des Pumpkolbens beziehungsweise des kombinierten Pump-Vakuum-Kolbens gelöst wird, wobei eine Druckfeder den Pumpkolben und den Vakuumkolben beziehungsweise den kombinierten Pump-Vakuum-Kolben in Richtung des Fußteils der Vorrichtung drückt,
d) wodurch die Monomerflüssigkeit durch die Verbindungsleitung (das Leitungsmittel) in den Innenraum der Kartusche in das Zementpulver gedrückt wird und durch die Bewegung des Vakuumkolbens beziehungsweise des kombinierten Pump-Vakuum-Kolbens Luft aus dem Innenraum der Kartusche gezogen wird,
e) dass nach vollständigen Transfer der Monomerflüssigkeit vom ersten Hohlraum in den Innenraum der Kartusche das Gemisch aus Zementpulver und Monomerflüssigkeit manuell durch Betätigung einer Mischstange gemischt wird,
f) dass dann die Mischstange nach oben gezogen wird,
g) die Mischstange an einer Sollbruchstelle abgebrochen wird und
h) dass dann die Kartusche vom Fußteil getrennt wird.

Im Folgenden werden weitere Ausführungsbeispiele der Erfindung anhand von neun schematisch dargestellten Figuren erläutert, ohne jedoch dabei die Erfindung zu beschränken. Dabei zeigt:
- Figur 1:: eine schematische perspektivische Ansicht einer erfindungsgemäßen Vorrichtung als Full-Prepacked-Mischsystem;
- Figur 2:: die Vorrichtung nach Figur 1 in einer Frontalansicht mit einer Schnittebene A-A;
- Figur 3:: die Vorrichtung nach den Figuren 1 und 2 in einer schematischen Querschnittansicht entsprechend der Schnittebene A nach Figur 2;
- Figur 4:: einen Teilbereich der Vorrichtung nach den Figuren 1, 2 und 3 in einer schematischen Querschnittansicht entsprechend einer Schnittebene parallel zu Schnittebene A-A nach Figur 2;
- Figur 5:: eine schematische perspektivische Ansicht einer alternativen zweiten erfindungsgemäßen Vorrichtung als Full-Prepacked-Mischsystem;
- Figur 6:: die Vorrichtung nach Figur 5 in einer schematischen Querschnittansicht;
- Figur 7:: eine schematische perspektivische Ansicht einer alternativen dritten erfindungsgemäßen Vorrichtung als Full-Prepacked-Mischsystem;
- Figur 8:: die Vorrichtung nach Figur 7 in einer schematischen Querschnittansicht; und
- Figur 9:: eine schematische Querschnittansicht einer alternativen vierten erfindungsgemäßen Vorrichtung als Full-Prepacked-Mischsystem in der das schematische Funktionsprinzip der Vorrichtung dargestellt ist.

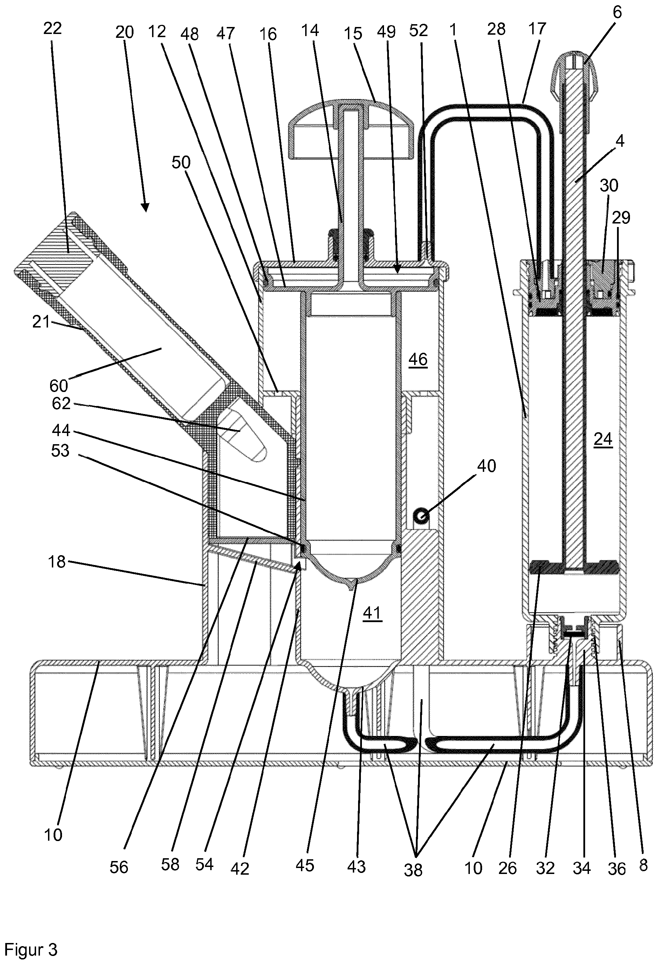

In den Querschnittansichten der Figuren 3, 4, 6, 8 und 9 sind geschnittene Flächen durch Schraffierungen kenntlich gemacht.

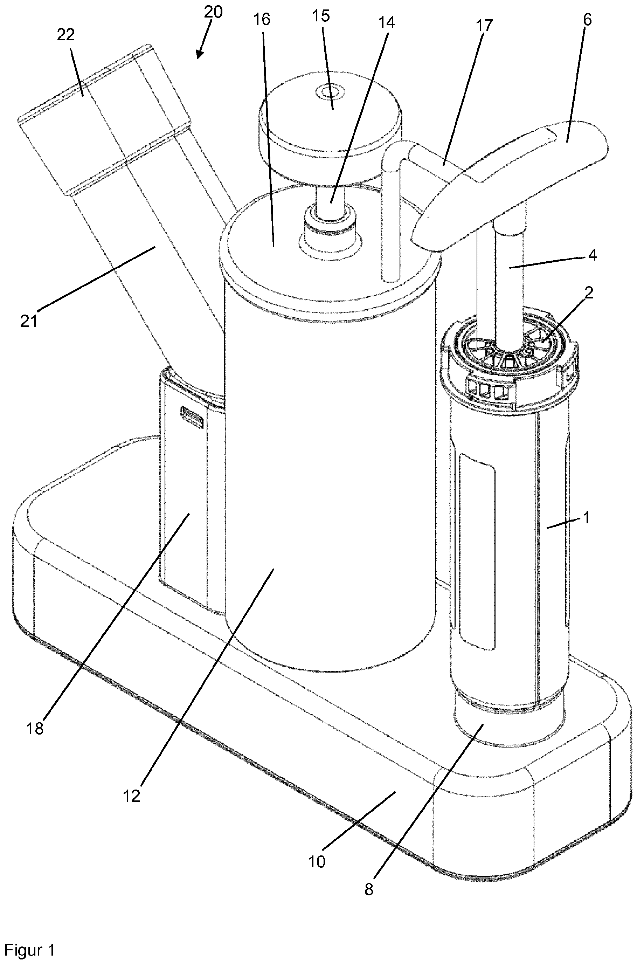

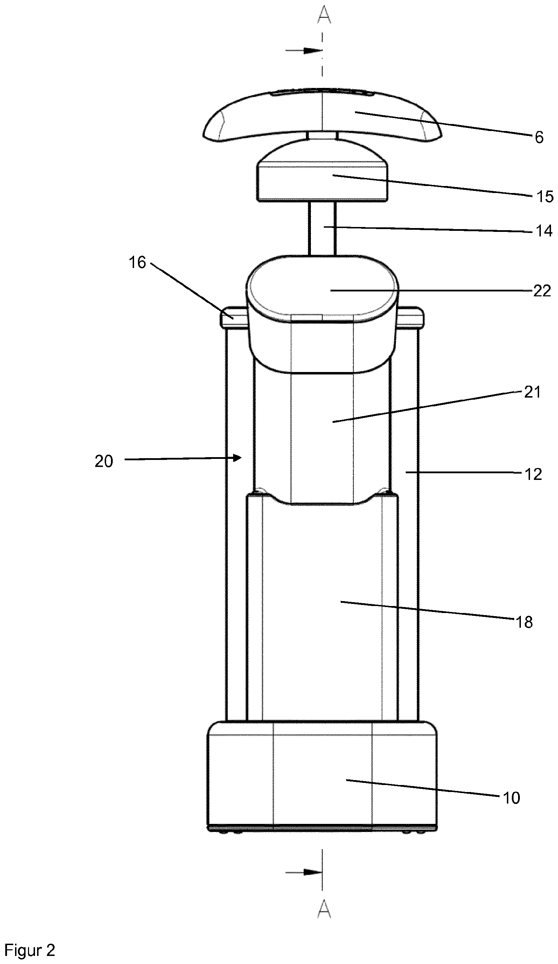

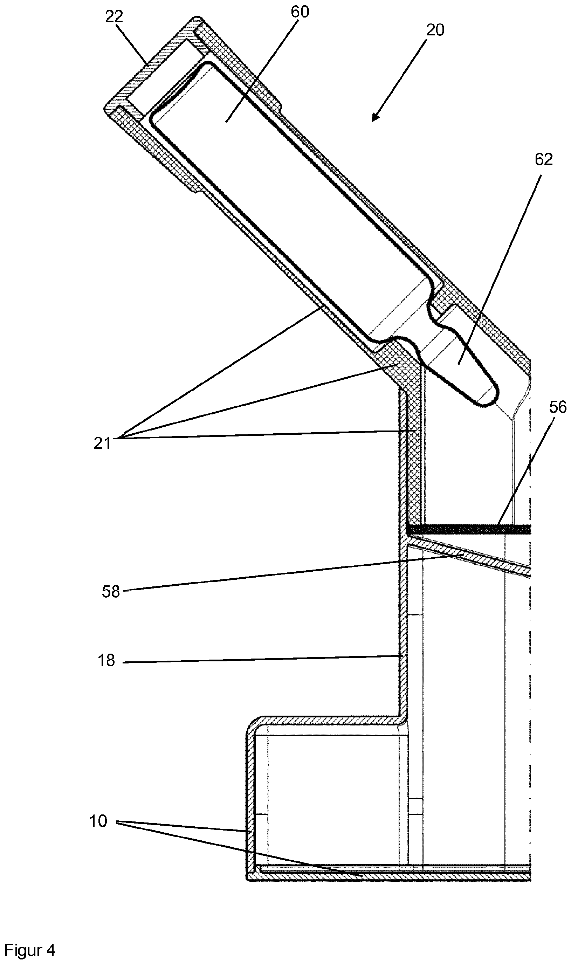

Die Figuren 1 bis 4 zeigen schematische Darstellungen einer ersten erfindungsgemäßen Vorrichtung, die zum Durchführen eines erfindungsgemäßen Verfahrens geeignet ist. Dabei ist in Figur 1 eine schematische perspektivische Ansicht einer erfindungsgemäßen Vorrichtung als Full-Prepacked-Mischsystem dargestellt, in Figur 2 die Vorrichtung nach Figur 1 in einer Frontalansicht mit einer Schnittebenen A-A, in Figur 3 die Vorrichtung nach den Figuren 1 und 2 in einer schematischen Querschnittansicht entsprechend der Schnittebene A-A nach Figur 2 und in Figur 4 ein Teilbereich dieser Vorrichtung nach den Figuren 1, 2 und 3 in einer schematischen Querschnittansicht entsprechend einer Schnittebene, die parallel zur Schnittebene A-A nach Figur 2 ist, dargestellt.

Die Vorrichtung umfasst eine Kartusche 1 in der ein Zementpulver als Ausgangskomponente des herzustellenden PMMA-Knochenzements enthalten ist. Auf der Oberseite (in den Figuren 1 und 3 oben) ist die Kartusche 1 mit einem Austragskolben 2 verschlossen. Der Austragskolben 2 kann mit den Wandungen der Kartusche 1 arretiert sein. Durch eine zentrische Durchführung in dem Austragskolben 2 ist eine Mischstange 4 geführt, die sich in die Kartusche 1 hinein und herausziehen lässt und die in dem Austragskolben 2 und damit in der Kartusche 1 drehbar gelagert ist. An dem oberen Ende der Mischstange 4 (in den Figuren 1, 2 und 3 oben) ist ein Griffstück 6 zur manuellen Bedienung der Mischstange 4 befestigt.

Im Bereich eines Stutzens 8 an einem Standfuß 10 der Vorrichtung ist die Kartusche 1 mit dem Standfuß 10 lösbar befestigt. Einteilig mit dem Standfuß 10 ausgeführt ist an dessen Oberseite ein oberer (äußerer) Hohlzylinder 12 angeordnet. Eine Stange 14 mit einer Drucktaste 15 zum Bedienen der Stange 14 ist durch eine Durchführung in einem Abschlussdeckel 16 durchgeführt. Der Abschlussdeckel 16 schließt den oberen Hohlzylinder 12 druckdicht nach oben ab. Hierzu ist in der Durchführung für die Stange 14 ein Dichtungsring vorgesehen, mit dem die Stange 14 gegen die Durchführung abgedichtet ist. In dem Austragskolben 2 ist ein Vakuumanschluss vorgesehen, an den eine Vakuumleitung 17 angeschlossen ist, die in das Innere des oberen Hohlzylinders 12 führt. Mit dem Vakuumanschluss kann das Innere der Kartusche 1 evakuiert werden und durch den Vakuumanschluss kann theoretisch auch Ethylenoxid zur Sterilisation des Inhalts der Kartusche 1 eingefüllt werden, wenn die Vakuumleitung 17 nicht an den Vakuumanschluss angeschlossen ist, beziehungsweise bevor die Vakuumleitung 17 an den Vakuumanschluss angeschlossen ist.

Neben dem Hohlzylinder 12 ist am Standfuß 10 noch ein Anschlussstutzen 18 vorgesehen, der ein Teil eines Anschlusses 20 für einen Monomer-Behälter 60 der Vorrichtung bildet. Der Anschluss 20 für den Monomer-Behälter 60 umfasst neben dem Anschlussstutzen 18 noch eine elastische Aufnahme 21 für den Monomer-Behälter 60 und einen Deckel 22, mit dem der Anschluss 20 für den Monomer-Behälter 60 nach außen abschließbar ist, nachdem der Monomer-Behälter 60 einsteckt wurde. Der Monomer-Behälter 60 ist im Inneren des Anschlusses 20 und damit in der Vorrichtung angeordnet. Der Monomer-Behälter 60 ist eine Glasampulle, die mit einer Monomerflüssigkeit als zweiter Komponente für den herzustellenden PMMA-Knochenzement gefüllt ist. Die elastische Aufnahme 21 besteht aus Gummi oder einem anderen elastischen Kunststoff.

Weitere Details zum Aufbau der Vorrichtung sind in den Figuren 3 und 4 zu erkennen. Das Innere der Kartusche 1 wird durch einen zylindrischen Innenraum 24 gebildet, in dem das Zementpulver als erste Komponente enthalten ist. Zudem ist im Innenraum 24 der Kartusche 1 eine Mischeinrichtung 26 bestehend aus mehreren Mischflügeln 26 angeordnet, die an der Mischstange 4 befestigt ist und die über die Mischstange 4 im Innenraum 24 der Kartusche 1 bewegt werden kann. Der Austragskolben 2 ist zweiteilig aufgebaut und besteht aus einem Sterilisationskolben 28 (in Figur 3 oberer Teil des Austragskolbens) und einem mit einer Dichtung 29 gegen die Innenwand der Innenraums 24 abgedichteten Dichtungskolben 30 (in Figur 3 unterer Teil des Austragskolbens). Der Dichtungskolben 30 weist eine gasdurchlässige aber für Pulver undurchlässige Porenscheibe auf, durch die der Innenraum 24 evakuierbar ist. Der Austragskolben 2 hat einen zylindrischen Außenumfang und schließt dicht mit den Wänden des Innenraums 24 ab. Der Austragskolben 2 kann im Innenraum 24 in Richtung einer Austragsöffnung am Boden des Innenraums 24 der Kartusche 1 vorgetrieben werden, die auf der dem Austragskolben 2 gegenüberliegenden Seite des Innenraums 24 der Kartusche 1 angeordnet ist.

Zum Anschließen der Kartusche 1 an den Standfuß 10 ist ein Standfuß-Anschluss 34 mit einem Außengewinde als Verbindung zur Kartusche 1 vorgesehen, in dem ein pulverundurchlässiger und flüssigkeitsdurchlässiger Filter 32 angeordnet ist. Auf der dem Austragskolben 2 gegenüberliegenden Seite des Innenraums 24 der Kartusche 1 ist ein zum Außengewinde des Standfuß-Anschlusses 34 passender Kartuschenanschluss 36 mit Innengewinde vorgesehen. Der Kartuschenanschluss 36 begrenzt die Austragsöffnung der Kartusche 1. Der Kartuschenanschluss 36 ist auf den Standfuß-Anschluss 34 aufgeschraubt und schließt mit diesem dicht ab.

Zwischen dem Hohlzylinder 12 und dem Innenraum 24 der Kartusche 1 ist eine Verbindungsleitung 38 vorgesehen, die den Hohlzylinder 12 mit dem Innenraum 24 der Kartusche 1 verbindet. An der Einmündung in den Innenraum 24 der Kartusche 1 ist der Filter 32 angeordnet, der verhindert, dass Zementpulver aus dem Innenraum 24 der Kartusche 1 in die Verbindungsleitung 38 eindringt. Die Verbindungsleitung 38 bildet eine Schlaufe 40 mit einem hohen Scheitelpunkt, um ein unkontrolliertes Durchfließen der Monomerflüssigkeit durch die Verbindungsleitung 38 in den Innenraum 24 der Kartusche 1 zu verhindern. Ein kleines Sichtfenster (nicht gezeigt) kann zur visuellen Kontrolle der Schlaufe 40 vorgesehen sein. Dazu muss die Verbindungsleitung 38 im Bereich der Schlaufe 40 transparent sein.

Der Stutzen 8, Standfuß 10 und der Anschlussstutzen 18 für den Monomer-Behälter 60 sind einteilig aus einem Kunststoff beispielsweise durch Spritzgießen hergestellt. Die Verbindungsleitung 38 mündet in einen Hohlraum 41 in einem unteren Hohlzylinder 42, wobei der untere (innere) Hohlzylinder 42 einen kleineren Innendurchmesser hat als der obere (äußere) Hohlzylinder 12. Der untere Hohlzylinder 42 ist an der Unterseite (in Figur 3 unten) durch einen trichterförmigen Boden 43 begrenzt, der sich nach unten stetig verjüngt. Hierdurch wird sichergestellt, dass die Monomerflüssigkeit vollständig aus dem unteren Hohlzylinder 42 abfließen beziehungsweise herausgedrückt werden kann. In dem unteren Hohlzylinder 42 ist ein innen passender Pumpkolben 44 angeordnet, der sich in das Innere des unteren Hohlzylinders 42 in Richtung des trichterförmigen Bodens 43 einpressen beziehungsweise eindrücken lässt (in Figur 3 nach unten).

Der Pumpkolben 44 ist durch einen Hohlkörper aus einem Kunststoff gebildet und an der unteren Fläche 45 als Negativ des Bodens 43 des unteren Hohlzylinders 42 geformt. Der Pumpkolben 44 ist mit einer Dichtung 53 gegen die Innenwand des unteren Hohlzylinders 42 abgedichtet und in Längsrichtung (in Figur 3 nach unten) beweglich im unteren Hohlzylinder 42 gelagert.

Im Inneren des oberen Hohlzylinders 12 ist ein offener Hohlraum 46 vorgesehen, in dem ein Vakuumkolben 47 axial beweglich angeordnet ist. Der Vakuumkolben 47 ist mit einer Dichtung 48 gegen die Innenwand des oberen Hohlzylinders 12 abgedichtet. Dadurch trennt der Vakuumkolben 47 nach oben einen oberen geschlossenen Hohlraum 49 ab, der durch den Abschlussdeckel 16, die Innenwand des oberen Hohlzylinders 12 und durch den Vakuumkolben 47 begrenzt ist. Im Gegensatz dazu sind Öffnungen 50 im Boden des offenen unteren Hohlraums 46 vorgesehen. Der obere geschlossene Hohlraum 49 ist über einen Anschluss 52 mit der Vakuumleitung 17 verbunden. Der Vakuumkolben 47 ist fest mit dem Pumpkolben 44 verbunden. Es ist bei der vorliegenden Ausführung auch möglich, den Vakuumkolben 47 und den Pumpkolben 44 einteilig auszuführen.

An einer Mantelfläche des unteren Hohlzylinders 42, direkt unterhalb der unteren Fläche 45 des Pumpkolbens 44, ist eine Einmündung 54 von dem Anschluss 20 für den Monomer-Behälter 60 in den unteren Hohlzylinder 42 beziehungsweise den Hohlraum 41 vorgesehen. Die Einmündung 54 bildet ein Leitungsmittel für die Monomerflüssigkeit, so dass die Einmündung 54 als Teil einer Verbindungsleitung 38, 40 für die Monomerflüssigkeit aufgefasst werden kann, in der der untere Hohlzylinder 42 angeordnet ist.

In dem Anschluss 20 für den Monomer-Behälter 60 ist ein Sieb 56 oder Filter 56 angeordnet, mit dem Bruchstücke und Teile des geöffneten Monomer-Behälters 60 abgefangen werden können. Im Anschlussstutzen 18 ist unterhalb des Siebs 56 beziehungsweise des Filters 56 eine geneigte Bodenfläche 58 vorgesehen, die in Richtung der Einmündung 54 geneigt ist. Dadurch kann die gesamte Monomerflüssigkeit aus dem Monomer-Behälter 60 durch die Einmündung 54 in den unteren Hohlzylinder 42 fließen.

Der Monomer-Behälter 60 ist eine Glasampulle 60 mit einem abbrechbaren Ampullenkopf 62 und einem aufbrechbaren Genick, wobei das Genick den Ampullenkopf 62 mit dem Körper der Ampulle 60 verbindet. Durch die Elastizität der Aufnahme 21 für den Monomer-Behälter 60 und durch die Verdickung der Aufnahme 21 im Bereich des Genicks kann durch Biegen der Aufnahme 21 mit dem Monomer-Behälter 60 darin der Kopf 62 des Monomer-Behälters 60 abgebrochen werden. Die Aufnahme 21 mit der entsprechenden Form, insbesondere mit der Verdickung im Bereich des Genicks, bildet dadurch ein Öffnungsmittel 21 zum Öffnen des Monomer-Behälters 60. Andere Öffnungsmittel zum Abscheren des Kopfs 62 des Monomer-Behälters 60 sind ebenfalls umsetzbar.

Ein erfindungsgemäßes Verfahren kann mit der Vorrichtung nach den Figuren 1 bis 4 beispielsweise wie folgt umgesetzt werden. Die Vorrichtung wird mit dem Standfuß 10 auf einem Tisch oder einer anderen passenden ebenen Unterlage aufgestellt. Der Monomer-Behälter 60 wird durch Knicken der elastischen Aufnahme 21 geöffnet, indem der Kopf 62 abgebrochen beziehungsweise aufgebrochen wird. Die Monomerflüssigkeit aus dem Monomer-Behälter 60 fließt durch das Sieb 54 beziehungsweise den Filter 54 wobei Bruchstücke des Monomer-Behälters 60 zurückgehalten werden. Durch die geneigte Bodenfläche 58 wird die Monomerflüssigkeit durch die Einmündung 54 in den unteren Hohlzylinder 42 beziehungsweise den Hohlraum 41 geleitet. Der freie Hohlraum 41 des unteren Hohlzylinders 42 wird vollständig mit der Monomerflüssigkeit gefüllt, da in dem Monomer-Behälter 60 mehr Monomerflüssigkeit enthalten ist, als der untere Hohlzylinder 42, beziehungsweise der Hohlraum 41 zusammen mit der Verbindungsleitung 38 bis zur Schlaufe 40 aufnehmen kann. Lufteinschlüsse entweichen durch die Einmündung 54, da diese am höchsten Punkt des durch den Hohlzylinder 42, den Boden 45 des Pumpkolbens 44 und die Verbindungsleitung 38 bis unterhalb der Schlaufe 40 begrenzten Raums angeordnet ist. Die Monomerflüssigkeit kann dabei nicht über den Scheitelpunkt der Schlaufe 40 fließen, da dieser deutlich oberhalb der der Einmündung 54 angeordnet ist und auch oberhalb des Flüssigkeitsspiegels der Monomerflüssigkeit im Anschluss 20 angeordnet ist, so dass die Monomerflüssigkeit daher nicht ohne Druck derart hoch steigen wird.

Anschließend wird über die Drucktaste 15 und damit über die Stange 14 vom Anwender ein manueller Druck in Richtung des Standfußes 10 ausgeübt. Dadurch wird der Vakuumkolben 47 im Hohlraum 46 nach unten gedrückt und der Pumpkolben 44 wird in den Hohlzylinder 42 beziehungsweise den Hohlraum 41 eingedrückt. Der Pumpkolben 44 kann vollständig oder teilweise in den Hohlzylinder 42 beziehungsweise den Hohlraum 41 eingedrückt werden, um die gewünschte Menge Monomerflüssigkeit aus dem Hohlzylinder 42 durch die Verbindungsleitung 38 in den Innenraum 24 der Kartusche 1 zu überführen beziehungsweise zu pressen. Die Menge der eingepressten Monomerflüssigkeit lässt sich durch unterschiedlich tiefes Einschieben des Pumpkolbens 44 in den Hohlzylinder 42 einstellen. Hierzu können Markierungen (nicht gezeigt) außen an der Stange 14 vorgesehen sein. Die Monomerflüssigkeit wird durch den Filter 32 in den Innenraum 24 der Kartusche 1 gepresst und steigt dort an und mischt sich mit dem Zementpulver, das in dem Innenraum 24 der Kartusche 1 gelagert ist.

Gleichzeitig wird durch die Bewegung des Vakuumkolbens 47 der Hohlraum 49 zwischen dem Vakuumkolben 47 und dem Abschlussdeckel 46 im oberen Hohlzylinder 12 vergrößert. Dadurch verringert sich der Gasdruck im Hohlraum 49. Der verringerte Druck wird mittels der Vakuumleitung 17 und den Vakuumanschluss des Dichtungskolbens 30 sowie durch den Porenfilter des Sterilisationskolbens 28 in den Innenraum 24 der Kartusche 1 geleitet. Dadurch wird der Innenraum 24 der Kartusche 1 mit der Bewegung des Vakuumkolbens 47 beziehungsweise der Kolben 44, 47 evakuiert. Da der Vakuumkolben 47 einen größeren Durchmesser hat als der Pumpkolben 44, wird weniger Volumen der Monomerflüssigkeit aus dem Hohlraum 41 in den Innenraum 24 der Kartusche 1 gedrückt, als Gas durch das sich im Hohlraum 49 vergrößernde Volumen aus dem Innenraum der Kartusche 1 evakuiert wird. Daher verringert sich der Druck im Innenraum der Kartusche 1 beim Eindrücken des Vakuumkolbens 47 und des Pumpkolbens 44.

Nachdem die gewünschte Menge Monomerflüssigkeit in den Innenraum 24 der Kartusche 1 eingefüllt wurde, wird durch Hineinschieben, Herausziehen und Drehen der Mischeinrichtung 26 beziehungsweise der Mischstange 4 mit Hilfe des Griffstücks 6 manuell die Monomerflüssigkeit und das Zementpulver gemischt und so der Zementteig beziehungsweise der PMMA-Knochenzement gemischt. Nach erfolgter Durchmischung wird die Mischstange 4 bis zum Anschlag aus dem Innenraum 24 der Kartusche 1 herausgezogen und abgebrochen, damit sie später nicht stört. Gegebenenfalls kann eine Arretierung des Austragskolbens 2 gelöst werden. Anschließend wird die Vakuumleitung 17 vom Vakuumanschluss des Dichtungskolbens 30 getrennt und die Kartusche 1 vom Standfuß 10 abgeschraubt. Ein Austragsrohr (nicht gezeigt) kann auf das Innengewinde am Kartuschenanschluss 36 geschraubt werden. Anschließend kann der fertig gemischte Knochenzement aus dem Innenraum 24 der Kartusche 1 durch die Austragsöffnung und das Austragsrohr durch Einpressen des Austragskolbens 2 ausgetrieben und appliziert werden.

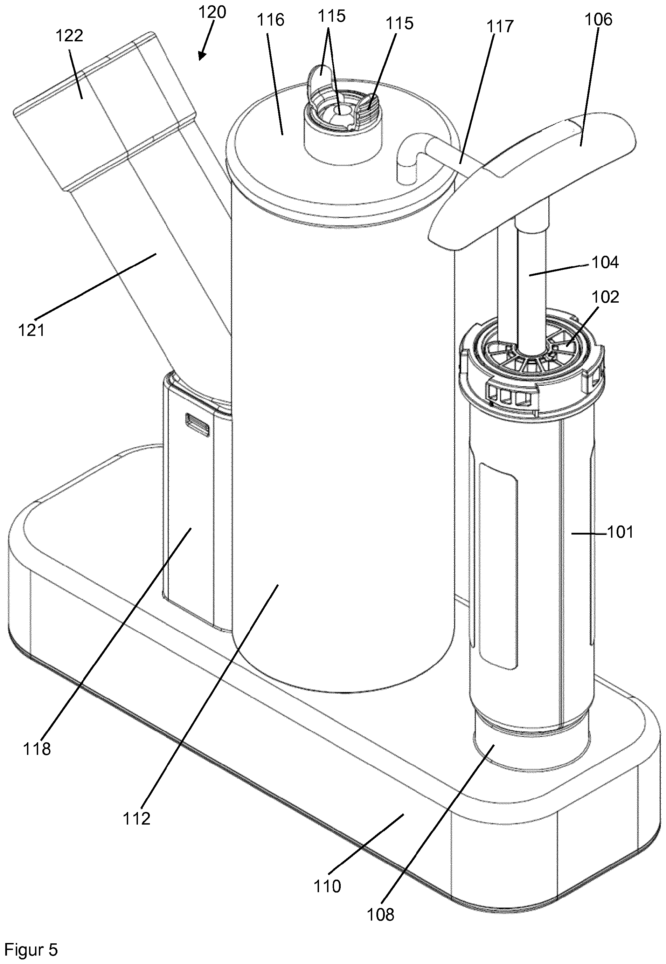

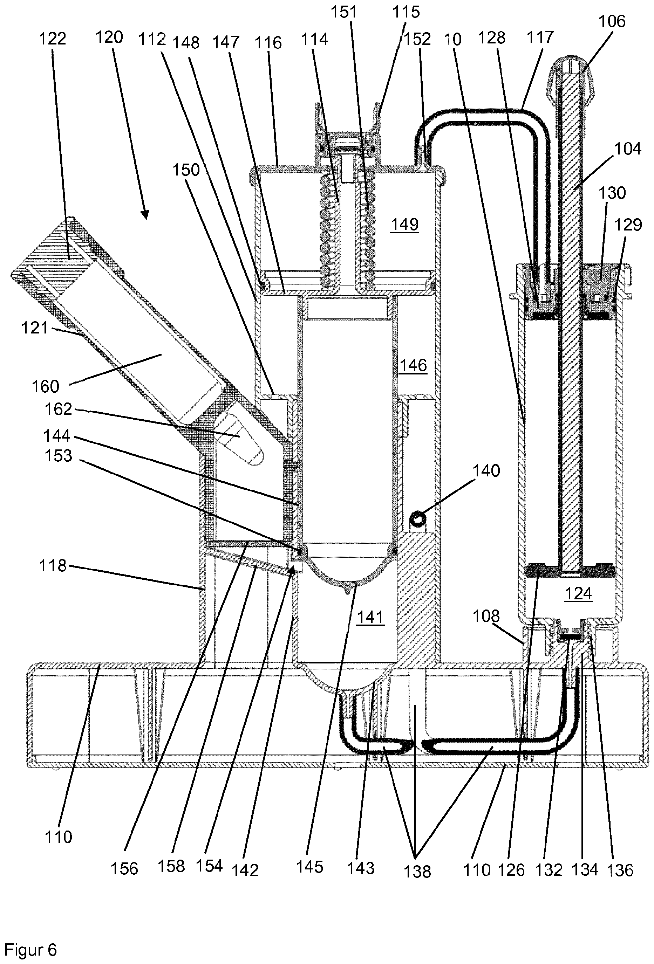

Die Figuren 5 und 6 zeigen schematische Darstellungen einer zweiten alternativen erfindungsgemäßen Vorrichtung, die zum Durchführen eines erfindungsgemäßen Verfahrens geeignet ist. Dabei ist in Figur 5 eine schematische perspektivische Ansicht der alternativen erfindungsgemäßen Vorrichtung als Full-Prepacked-Mischsystem dargestellt und in Figur 6 die Vorrichtung nach Figur 5 in einer schematischen Querschnittansicht dargestellt.

Die Vorrichtung umfasst eine Kartusche 101 in der ein Zementpulver als Ausgangskomponente des herzustellenden PMMA-Knochenzements enthalten ist. Auf der Oberseite (in den Figuren 5 und 6 oben) ist die Kartusche 101 mit einem Austragskolben 102 verschlossen. Der Austragskolben 102 kann mit den Wandungen der Kartusche 101 arretiert sein. Durch eine zentrische Durchführung in dem Austragskolben 102 ist eine Mischstange 104 geführt, die sich in die Kartusche 101 hinein und herausziehen lässt und die in dem Austragskolben 102 und damit in der Kartusche 101 drehbar gelagert ist. An dem oberen Ende der Mischstange 104 (in den Figuren 5 und 6 oben) ist ein Griffstück 106 zur manuellen Bedienung der Mischstange 104 befestigt.

Im Bereich eines Stutzens 108 an einem Standfuß 110 der Vorrichtung ist die Kartusche 101 mit dem Standfuß 110 lösbar befestigt. Einteilig mit dem Standfuß 110 ausgeführt ist an dessen Oberseite ein oberer (äußerer) Hohlzylinder 112 angeordnet. Eine arretierte Stange 114 mit einer Sicherung 115 zum Entarretieren der Stange 114 beziehungsweise zum Auslösen der Vorrichtung ist durch eine Durchführung in einem Abschlussdeckel 116 durchgeführt. Der Abschlussdeckel 116 schließt den oberen Hohlzylinder 112 druckdicht nach oben ab. Hierzu ist in einem Sitz der Sicherung 115 ein Dichtungsring vorgesehen, mit dem die Sicherung 115 gegen den Sitz abgedichtet ist.

In dem Austragskolben 102 ist ein Vakuumanschluss vorgesehen, an den eine Vakuumleitung 117 angeschlossen ist, die in das Innere des oberen Hohlzylinders 112 führt. Mit dem Vakuumanschluss kann das Innere der Kartusche 101 evakuiert werden und durch den Vakuumanschluss kann theoretisch auch Ethylenoxid zur Sterilisation des Inhalts der Kartusche 101 eingefüllt werden, wenn die Vakuumleitung 117 nicht an den Vakuumanschluss angeschlossen ist, beziehungsweise bevor die Vakuumleitung 117 an den Vakuumanschluss angeschlossen ist.

Neben dem Hohlzylinder 112 ist am Standfuß 110 noch ein Anschlussstutzen 118 vorgesehen, der ein Teil eines Anschlusses 120 für einen Monomer-Behälter 160 der Vorrichtung bildet. Der Anschluss 120 für den Monomer-Behälter 160 umfasst neben dem Anschlussstutzen 118 noch eine elastische Aufnahme 121 für den Monomer-Behälter 160 und einen Deckel 122, mit dem der Anschluss 120 für den Monomer-Behälter 160 nach außen abschließbar ist, nachdem der Monomer-Behälter 160 einsteckt wurde. Der Monomer-Behälter 160 ist im Inneren des Anschlusses 120 und damit in der Vorrichtung angeordnet. Der Monomer-Behälter 160 ist eine Glasampulle, die mit einer Monomerflüssigkeit als zweiter Komponente für den herzustellenden PMMA-Knochenzement gefüllt ist. Die elastische Aufnahme 121 besteht aus Gummi oder einem anderen elastischen Kunststoff.

Weitere Details zum Aufbau der Vorrichtung sind in Figur 6 zu erkennen. Das Innere der Kartusche 101 wird durch einen zylindrischen Innenraum 124 gebildet, in dem das Zementpulver als erste Komponente enthalten ist. Zudem ist im Innenraum 124 der Kartusche 101 eine Mischeinrichtung 126 bestehend aus mehreren Mischflügeln 126 angeordnet, die an der Mischstange 104 befestigt ist und die über die Mischstange 104 im Innenraum 124 der Kartusche 101 bewegt werden kann. Der Austragskolben 102 ist zweiteilig aufgebaut und besteht aus einem Sterilisationskolben 128 (in Figur 6 oberer Teil des Austragskolbens) und einem mit einer Dichtung 129 gegen die Innenwand der Innenraums 124 abgedichteten Dichtungskolben 130 (in Figur 6 unterer Teil des Austragskolbens). Der Dichtungskolben 130 weist eine gasdurchlässige aber für Pulver undurchlässige Porenscheibe auf, durch die der Innenraum 124 evakuierbar ist. Der Austragskolben 102 hat einen zylindrischen Außenumfang und schließt dicht mit den Wänden des Innenraums 124 ab. Der Austragskolben 102 kann im Innenraum 124 in Richtung einer Austragsöffnung am Boden des Innenraums 124 der Kartusche 101 vorgetrieben werden, die auf der dem Austragskolben 102 gegenüberliegenden Seite des Innenraums 124 der Kartusche 101 angeordnet ist.

Zum Anschließen der Kartusche 101 an den Standfuß 110 ist ein Standfuß-Anschluss 134 mit einem Außengewinde als Verbindung zur Kartusche 101 vorgesehen, in dem ein pulverundurchlässiger und flüssigkeitsdurchlässiger Filter 132 angeordnet ist. Auf der dem Austragskolben 102 gegenüberliegenden Seite des Innenraums 124 der Kartusche 101 ist ein zum Außengewinde des Standfuß-Anschlusses 134 passender Kartuschenanschluss 136 mit Innengewinde vorgesehen. Der Kartuschenanschluss 136 begrenzt die Austragsöffnung der Kartusche 101. Der Kartuschenanschluss 136 ist auf den Standfuß-Anschluss 134 aufgeschraubt und schließt mit diesem dicht ab.

Zwischen dem Hohlzylinder 112 und dem Innenraum 124 der Kartusche 101 ist eine Verbindungsleitung 138 vorgesehen, die den Hohlzylinder 112 mit dem Innenraum 124 der Kartusche 101 verbindet. An der Einmündung in den Innenraum 124 der Kartusche 101 ist der Filter 132 angeordnet, der verhindert, dass Zementpulver aus dem Innenraum 124 der Kartusche 101 in die Verbindungsleitung 138 eindringt. Die Verbindungsleitung 138 bildet eine Schlaufe 140 mit einem hohen Scheitelpunkt, um ein unkontrolliertes Durchfließen der Monomerflüssigkeit durch die Verbindungsleitung 138 in den Innenraum 124 der Kartusche 101 zu verhindern. Ein kleines Sichtfenster (nicht gezeigt) kann zur visuellen Kontrolle der Schlaufe 140 vorgesehen sein. Dazu muss die Verbindungsleitung 138 im Bereich der Schlaufe 140 transparent sein.

Der Stutzen 108, Standfuß 110 und der Anschlussstutzen 118 für den Monomer-Behälter 160 sind einteilig aus einem Kunststoff beispielsweise durch Spritzgießen hergestellt. Die Verbindungsleitung 138 mündet in einen Hohlraum 141 in einem unteren Hohlzylinder 142, wobei der untere (innere) Hohlzylinder 142 einen kleineren Innendurchmesser hat als der obere (äußere) Hohlzylinder 112. Der untere Hohlzylinder 142 ist an der Unterseite (in Figur 6 unten) durch einen trichterförmigen Boden 143 begrenzt, der sich nach unten stetig verjüngt. Hierdurch wird sichergestellt, dass die Monomerflüssigkeit vollständig aus dem unteren Hohlzylinder 142 abfließen beziehungsweise herausgedrückt werden kann. In dem unteren Hohlzylinder 142 ist ein innen passender Pumpkolben 144 angeordnet, der sich in das Innere des unteren Hohlzylinders 142 in Richtung des trichterförmigen Bodens 143 einpressen beziehungsweise eindrücken lässt (in Figur 6 nach unten).

Der Pumpkolben 144 ist durch einen Hohlkörper aus einem Kunststoff gebildet und an der unteren Fläche 145 als Negativ des Bodens 143 des unteren Hohlzylinders 142 geformt. Der Pumpkolben 144 ist mit einer Dichtung 153 gegen die Innenwand des unteren Hohlzylinders 142 abgedichtet und in Längsrichtung (in Figur 6 nach unten) beweglich im unteren Hohlzylinder 142 gelagert.

Im Inneren des oberen Hohlzylinders 112 ist ein offener Hohlraum 146 vorgesehen, in dem ein Vakuumkolben 147 axial beweglich angeordnet ist. Der Vakuumkolben 147 ist mit einer Dichtung 148 gegen die Innenwand des oberen Hohlzylinders 112 abgedichtet. Dadurch trennt der Vakuumkolben 147 nach oben einen oberen geschlossenen Hohlraum 149 ab, der durch den Abschlussdeckel 116, die Innenwand des oberen Hohlzylinders 112, die Sicherung 115 und durch den Vakuumkolben 147 begrenzt ist. Im Gegensatz dazu sind Öffnungen 150 im Boden des offenen unteren Hohlraums 146 vorgesehen. Im Inneren des Hohlraums 149 des oberen Hohlzylinders 112 ist eine gespannte Druckfeder 151 vorgesehen, die um die Stange 114 herum liegend angeordnet ist. Die Spannung der Druckfeder 151 wird durch die mit dem Abschlussdeckel 116 arretierte Stange 114 zwischen dem Abschlussdeckel 116 und der Oberseite des Vakuumkolbens 147 gehalten. Der obere geschlossene Hohlraum 149 ist über einen Anschluss 152 mit der Vakuumleitung 117 verbunden. Der Vakuumkolben 147 ist fest mit dem Pumpkolben 144 verbunden. Es ist bei der vorliegenden Ausführung auch möglich, den Vakuumkolben 147 und den Pumpkolben 144 einteilig auszuführen.

An einer Mantelfläche des unteren Hohlzylinders 142, direkt unterhalb der unteren Fläche 145 des Pumpkolbens 144, ist eine Einmündung 154 von dem Anschluss 120 für den Monomer-Behälter 160 in den unteren Hohlzylinder 142 beziehungsweise den Hohlraum 141 vorgesehen. Die Einmündung 154 bildet ein Leitungsmittel für die Monomerflüssigkeit, so dass die Einmündung 154 als Teil einer Verbindungsleitung 138, 140 für die Monomerflüssigkeit aufgefasst werden kann, in der der untere Hohlzylinder 142 angeordnet ist.

In dem Anschluss 120 für den Monomer-Behälter 160 ist ein Sieb 156 oder Filter 156 angeordnet, mit dem Bruchstücke und Teile des geöffneten Monomer-Behälters 160 abgefangen werden können. Im Anschlussstutzen 118 ist unterhalb des Siebs 156 beziehungsweise des Filters 156 eine geneigte Bodenfläche 158 vorgesehen, die in Richtung der Einmündung 154 geneigt ist. Dadurch kann die gesamte Monomerflüssigkeit aus dem Monomer-Behälter 160 durch die Einmündung 154 in den unteren Hohlzylinder 142 fließen.

Der Monomer-Behälter 160 ist eine Glasampulle 160 mit einem abbrechbaren Ampullenkopf 162 und einem aufbrechbaren Genick, wobei das Genick den Ampullenkopf 162 mit dem Körper der Ampulle 160 verbindet. Durch die Elastizität der Aufnahme 121 für den Monomer-Behälter 160 und durch die Verdickung der Aufnahme 121 im Bereich des Genicks kann durch Biegen der Aufnahme 121 mit dem Monomer-Behälter 160 darin der Kopf 162 des Monomer-Behälters 160 abgebrochen werden. Die Aufnahme 121 mit der entsprechenden Form, insbesondere mit der Verdickung im Bereich des Genicks, bildet dadurch ein Öffnungsmittel 121 zum Öffnen des Monomer-Behälters 160. Andere Öffnungsmittel zum Abscheren des Kopfs 162 des Monomer-Behälters 160 sind ebenfalls umsetzbar.

Ein erfindungsgemäßes Verfahren kann mit der alternativen zweiten Vorrichtung nach den Figuren 5 und 6 beispielsweise wie folgt umgesetzt werden. Die Vorrichtung wird mit dem Standfuß 110 auf einem Tisch oder einer anderen passenden ebenen Unterlage aufgestellt. Der Monomer-Behälter 160 wird durch Knicken der elastischen Aufnahme 121 geöffnet, indem der Kopf 162 abgebrochen beziehungsweise aufgebrochen wird. Die Monomerflüssigkeit aus dem Monomer-Behälter 160 fließt durch das Sieb 156 beziehungsweise den Filter 156 wobei Bruchstücke des Monomer-Behälters 160 zurückgehalten werden. Durch die geneigte Bodenfläche 158 wird die Monomerflüssigkeit durch die Einmündung 154 in den unteren Hohlzylinder 142 beziehungsweise den Hohlraum 141 geleitet. Der freie Hohlraum 141 des unteren Hohlzylinders 142 wird vollständig mit der Monomerflüssigkeit gefüllt, da in dem Monomer-Behälter 160 mehr Monomerflüssigkeit enthalten ist, als der untere Hohlzylinder 142, beziehungsweise der Hohlraum 141 zusammen mit der Verbindungsleitung 138 bis zur Schlaufe 140 aufnehmen kann. Lufteinschlüsse entweichen durch die Einmündung 154, da diese am höchsten Punkt des durch den Hohlzylinder 142, den Boden 145 des Pumpkolbens 144 und die Verbindungsleitung 138 bis unterhalb der Schlaufe 140 begrenzten Raums angeordnet ist. Die Monomerflüssigkeit kann dabei nicht über den Scheitelpunkt der Schlaufe 140 fließen, da dieser deutlich oberhalb der der Einmündung 154 angeordnet ist und auch oberhalb des Flüssigkeitsspiegels der Monomerflüssigkeit im Anschluss 120 angeordnet ist, so dass die Monomerflüssigkeit daher nicht ohne Druck derart hoch steigen wird.

Durch Zusammenpressen zweier flacher Griffhebel, die an der Sicherung 115 angeordnet sind und die aus dem Sitz im Abschlussdeckel 116 herausragen, kann die Sicherung 115 in den Sitz nach unten gedrückt werden. Hierdurch schiebt sich die Sicherung 115 auf das obere Ender der Stange 114 und mehrere am oberen Ende der Stange 114 angeordnete Rasthaken werden verformt. Dadurch wird die Stange 114 frei beweglich und dadurch augenblicklich die Stange 114 mit dem Vakuumkolben 147 und dem Pumpkolben 144 von der gespannten Druckfeder 151 in Richtung des Standfußes 110 beschleunigt.

Mit der Druckfeder 151 wird der Vakuumkolben 147 im Hohlraum 146 nach unten gedrückt. Die Monomerflüssigkeit aus dem Hohlraum 141 wird durch die Verbindungsleitung 138 und den Filter 132 in den Innenraum 124 der Kartusche 101 gepresst und steigt dort an und mischt sich mit dem Zementpulver, das in dem Innenraum 124 der Kartusche 101 gelagert ist.

Gleichzeitig wird durch die Bewegung des Vakuumkolbens 147 der Hohlraum 149 zwischen dem Vakuumkolben 147 und dem Abschlussdeckel 146 im oberen Hohlzylinder 112 vergrößert. Dadurch verringert sich der Gasdruck im Hohlraum 149. Der verringerte Druck wird mittels der Vakuumleitung 117 und den Vakuumanschluss des Dichtungskolbens 130 sowie durch den Porenfilter des Sterilisationskolbens 128 in den Innenraum 124 der Kartusche 101 geleitet. Dadurch wird der Innenraum 124 der Kartusche 101 mit der Bewegung des Vakuumkolbens 147 beziehungsweise der Kolben 144, 147 evakuiert. Da der Vakuumkolben 147 einen größeren Durchmesser hat als der Pumpkolben 144, wird weniger Volumen der Monomerflüssigkeit aus dem Hohlraum 141 in den Innenraum 124 der Kartusche 101 gedrückt, als Gas durch das sich im Hohlraum 149 vergrößernde Volumen aus dem Innenraum der Kartusche 101 evakuiert wird. Daher verringert sich der Druck im Innenraum der Kartusche 101 beim Eindrücken des Vakuumkolbens 147 und des Pumpkolbens 144.

Nachdem die gewünschte Menge Monomerflüssigkeit in den Innenraum 124 der Kartusche 101 eingefüllt wurde, wird durch Hineinschieben, Herausziehen und Drehen der Mischeinrichtung 126 beziehungsweise der Mischstange 104 mit Hilfe des Griffstücks 6 manuell die Monomerflüssigkeit und das Zementpulver gemischt und so der Zementteig beziehungsweise der PMMA-Knochenzement gemischt. Nach erfolgter Durchmischung wird die Mischstange 104 bis zum Anschlag aus dem Innenraum 124 der Kartusche 101 herausgezogen und abgebrochen, damit sie später nicht stört. Gegebenenfalls kann eine Arretierung des Austragskolbens 102 gelöst werden. Anschließend wird die Vakuumleitung 117 vom Vakuumanschluss des Dichtungskolbens 130 getrennt und die Kartusche 101 vom Standfuß 110 abgeschraubt. Ein Austragsrohr (nicht gezeigt) kann auf das Innengewinde am Kartuschenanschluss 136 geschraubt werden. Anschließend kann der fertig gemischte Knochenzement aus dem Innenraum 124 der Kartusche 101 durch die Austragsöffnung und das Austragsrohr durch Einpressen des Austragskolbens 102 ausgetrieben und appliziert werden.

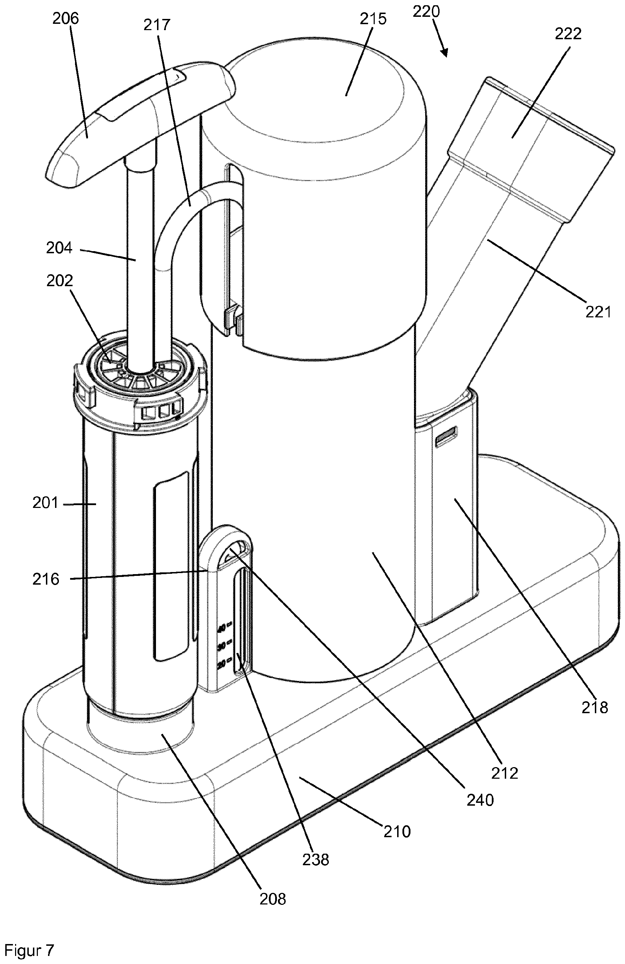

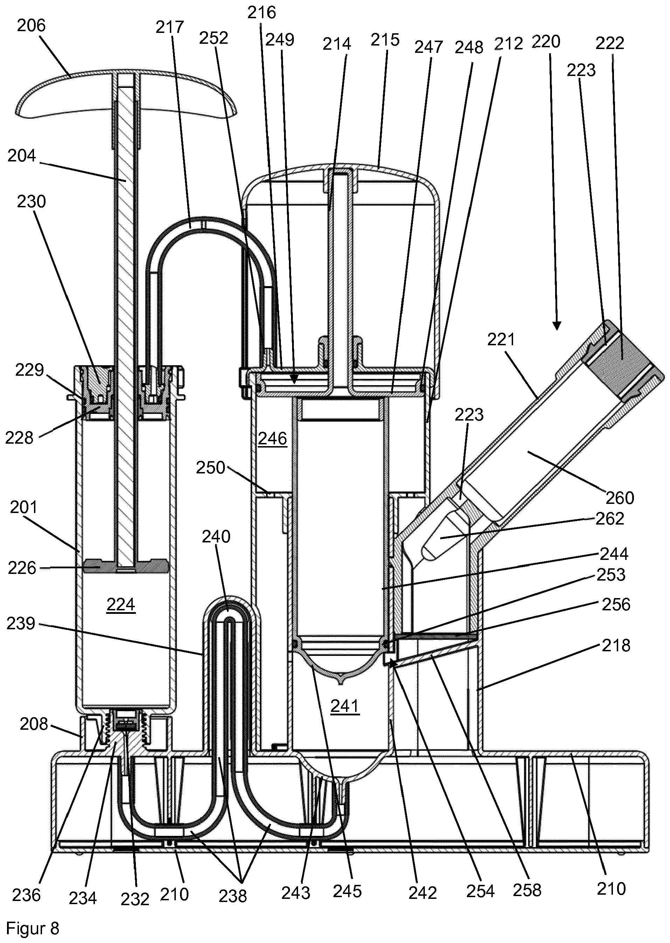

Die Figuren 7 und 8 zeigen schematische Darstellungen einer dritten alternativen erfindungsgemäßen Vorrichtung, die zum Durchführen eines erfindungsgemäßen Verfahrens geeignet ist. Dabei ist in Figur 7 eine schematische perspektivische Ansicht der alternativen erfindungsgemäßen Vorrichtung als Full-Prepacked-Mischsystem dargestellt und in Figur 8 die Vorrichtung nach Figur 7 in einer schematischen Querschnittansicht dargestellt.

Die dritte alternative Vorrichtung ist ähnlich wie die erste Vorrichtung nach den Figuren 1 bis 4 und die zweite alternative Vorrichtung nach den Figuren 5 und 6 aufgebaut und umfasst eine Kartusche 201 in der ein Zementpulver als erste Ausgangskomponente des herzustellenden PMMA-Knochenzements enthalten ist. Auf der Oberseite (in den Figuren 7 und 8 oben) ist die Kartusche 201 mit einem Austragskolben 202 verschlossen. Der Austragskolben 202 kann mit den Wandungen der Kartusche 201 arretiert sein. Durch eine zentrische Durchführung in dem Austragskolben 202 ist eine Mischstange 204 geführt, die sich in die Kartusche 201 hinein und herausziehen lässt und die in dem Austragskolben 202 und damit in der Kartusche 201 drehbar gelagert ist. An dem oberen Ende der Mischstange 204 (in den Figuren 7 und 8 oben) ist ein Griffstück 206 zur manuellen Bedienung der Mischstange 204 befestigt.

Im Bereich eines Stutzens 208 an einem Standfuß 210 der Vorrichtung ist die Kartusche 201 mit dem Standfuß 210 lösbar befestigt. Einteilig mit dem Standfuß 210 ausgeführt ist an dessen Oberseite ein oberer (äußerer) Hohlzylinder 212 angeordnet. Eine Stange 214 mit einer Haube 215 zum Bedienen der Stange 214 ist durch eine Durchführung in einem Abschlussdeckel 216 durchgeführt. Der Abschlussdeckel 216 schließt den oberen Hohlzylinder 212 druckdicht nach oben ab. Hierzu ist in der Durchführung für die Stange 214 ein Dichtungsring vorgesehen, mit dem die Stange 214 gegen die Durchführung abgedichtet ist.

In dem Austragskolben 202 ist ein Vakuumanschluss vorgesehen, an den eine Vakuumleitung 217 angeschlossen ist, die in das Innere des oberen Hohlzylinders 212 führt. Mit dem Vakuumanschluss kann das Innere der Kartusche 201 evakuiert werden und durch den Vakuumanschluss kann theoretisch auch Ethylenoxid zur Sterilisation des Inhalts der Kartusche 201 eingefüllt werden, wenn die Vakuumleitung 217 nicht an den Vakuumanschluss angeschlossen ist, beziehungsweise bevor die Vakuumleitung 217 an den Vakuumanschluss angeschlossen ist.

Neben dem Hohlzylinder 212 ist am Standfuß 210 noch ein Anschlussstutzen 218 vorgesehen, der ein Teil eines Anschlusses 220 für einen Monomer-Behälter 260 der Vorrichtung bildet. Der Anschluss 220 für den Monomer-Behälter 260 umfasst neben dem Anschlussstutzen 218 noch eine elastische Aufnahme 221 für den Monomer-Behälter 260 und einen Deckel 222, mit dem der Anschluss 220 für den Monomer-Behälter 260 nach außen abschließbar ist, nachdem der Monomer-Behälter 260 einsteckt wurde. Der Monomer-Behälter 260 ist im Inneren des Anschlusses 220 und damit in der Vorrichtung angeordnet. Der Monomer-Behälter 260 ist eine Glasampulle, die mit einer Monomerflüssigkeit als zweiter Komponente für den herzustellenden PMMA-Knochenzement gefüllt ist. Die elastische Aufnahme 221 besteht aus Gummi oder einem anderen elastischen Kunststoff. In dem Deckel 222 und in der Verdickung der Aufnahme 220 sind mehrere Belüftungsdurchgänge 223 angeordnet. Die Belüftungsdurchgänge 223 dienen dazu, dass die Monomerflüssigkeit gut aus der geöffneten Ampulle 260 beziehungsweise dem geöffneten Monomer-Behälter 260 ausfließen kann. Die Belüftungsdurchgänge 223 verhindern dabei das Entstehen eines Unterdrucks im Bereich des Deckels 222 des Monomer-Behälters 220, der dem Fluss der Monomerflüssigkeit entgegenwirken würde.

Weitere Details zum Aufbau der Vorrichtung sind in Figur 8 zu erkennen. Das Innere der Kartusche 201 wird durch einen zylindrischen Innenraum 224 gebildet, in dem das Zementpulver als erste Komponente enthalten ist. Zudem ist im Innenraum 224 der Kartusche 201 eine Mischeinrichtung 226 bestehend aus mehreren Mischflügeln 226 angeordnet, die an der Mischstange 204 befestigt ist und die über die Mischstange 204 im Innenraum 224 der Kartusche 201 bewegt werden kann. Der Austragskolben 202 ist zweiteilig aufgebaut und besteht aus einem Sterilisationskolben 228 (in Figur 8 oberer Teil des Austragskolbens) und einem mit einer Dichtung 229 gegen die Innenwand der Innenraums 224 abgedichteten Dichtungskolben 230 (in Figur 8 unterer Teil des Austragskolbens). Der Dichtungskolben 230 weist eine gasdurchlässige aber für Pulver undurchlässige Porenscheibe auf, durch die der Innenraum 224 evakuierbar ist. Der Austragskolben 202 hat einen zylindrischen Außenumfang und schließt dicht mit den Wänden des Innenraums 224 ab. Der Austragskolben 202 kann im Innenraum 224 in Richtung einer Austragsöffnung am Boden des Innenraums 224 der Kartusche 201 vorgetrieben werden, die auf der dem Austragskolben 202 gegenüberliegenden Seite des Innenraums 224 der Kartusche 201 angeordnet ist.

Zum Anschließen der Kartusche 201 an den Standfuß 210 ist ein Standfuß-Anschluss 234 mit einem Außengewinde als Verbindung zur Kartusche 201 vorgesehen, in dem ein pulverundurchlässiger und flüssigkeitsdurchlässiger Filter 232 angeordnet ist. Auf der dem Austragskolben 202 gegenüberliegenden Seite des Innenraums 224 der Kartusche 201 ist ein zum Außengewinde des Standfuß-Anschlusses 234 passender Kartuschenanschluss 236 mit Innengewinde vorgesehen. Der Kartuschenanschluss 236 begrenzt die Austragsöffnung der Kartusche 201. Der Kartuschenanschluss 236 ist auf den Standfuß-Anschluss 234 aufgeschraubt und schließt mit diesem dicht ab.

Zwischen dem Hohlzylinder 212 und dem Innenraum 224 der Kartusche 201 ist eine Verbindungsleitung 238 vorgesehen, die den Hohlzylinder 212 mit dem Innenraum 224 der Kartusche 201 verbindet. An der Einmündung in den Innenraum 224 der Kartusche 201 ist der Filter 232 angeordnet, der verhindert, dass Zementpulver aus dem Innenraum 224 der Kartusche 201 in die Verbindungsleitung 238 eindringt. Die Verbindungsleitung 238 ist bereichsweise in einem Gehäuse 239 geführt, in dem Sichtfenster enthalten sind. Die Verbindungsleitung 238 bildet in dem Gehäuse 239 eine Schlaufe 240 mit einem hohen Scheitelpunkt, um ein unkontrolliertes Durchfließen der Monomerflüssigkeit durch die Verbindungsleitung 238 in den Innenraum 224 der Kartusche 201 zu verhindern. Die Sichtfenster im Gehäuse 239 sind zur visuellen Kontrolle der Schlaufe 240 vorgesehen. Zudem kann die Standhöhe der Monomerflüssigkeit in der Verbindungsleitung 238 bestimmt werden. Hierzu ist eine Skala an einem der Sichtfenster angebracht. Damit die Standhöhe gesehen werden kann, muss die Verbindungsleitung 238 im Bereich der Schlaufe 240 transparent sein.

Der Stutzen 208, Standfuß 210, das Gehäuse 239 und der Anschlussstutzen 218 für den Monomer-Behälter 260 sind einteilig aus einem Kunststoff beispielsweise durch Spritzgießen hergestellt. Die Verbindungsleitung 238 mündet in einen Hohlraum 241 in einem unteren Hohlzylinder 242, wobei der untere (innere) Hohlzylinder 242 einen kleineren Innendurchmesser hat als der obere (äußere) Hohlzylinder 212. Der untere Hohlzylinder 242 ist an der Unterseite (in Figur 8 unten) durch einen trichterförmigen Boden 243 begrenzt, der sich nach unten stetig verjüngt. Hierdurch wird sichergestellt, dass die Monomerflüssigkeit vollständig aus dem unteren Hohlzylinder 242 abfließen beziehungsweise herausgedrückt werden kann. In dem unteren Hohlzylinder 242 ist ein innen passender Pumpkolben 244 angeordnet, der sich in das Innere des unteren Hohlzylinders 242 in Richtung des trichterförmigen Bodens 243 einpressen beziehungsweise eindrücken lässt (in Figur 8 nach unten).

Der Pumpkolben 244 ist durch einen Hohlkörper aus einem Kunststoff gebildet und an der unteren Fläche 245 als Negativ des Bodens 243 des unteren Hohlzylinders 242 geformt. Der Pumpkolben 244 ist mit einer Dichtung 253 gegen die Innenwand des unteren Hohlzylinders 242 abgedichtet und in Längsrichtung (in Figur 8 nach unten) beweglich im unteren Hohlzylinder 242 gelagert.

Im Inneren des oberen Hohlzylinders 212 ist ein offener Hohlraum 246 vorgesehen, in dem ein Vakuumkolben 247 axial beweglich angeordnet ist. Der Vakuumkolben 247 ist mit einer Dichtung 248 gegen die Innenwand des oberen Hohlzylinders 212 abgedichtet. Dadurch trennt der Vakuumkolben 247 nach oben einen oberen geschlossenen Hohlraum 249 ab, der durch den Abschlussdeckel 216, die Innenwand des oberen Hohlzylinders 212, die Sicherung 215 und durch den Vakuumkolben 247 begrenzt ist. Im Gegensatz dazu sind Öffnungen 250 im Boden des offenen unteren Hohlraums 246 vorgesehen. Der obere geschlossene Hohlraum 249 ist über einen Anschluss 252 mit der Vakuumleitung 217 verbunden. In der Haube 215 ist ein senkrechter Schlitz vorgesehen, durch den die flexible Vakuumleitung 217 geführt ist. Wenn die Haube 215 nach Unten in Richtung Standfuß 210 gedrückt wird gleitet die Vakuumleitung 217 in diesem Schlitz. Der Vakuumkolben 247 ist fest mit dem Pumpkolben 244 verbunden. Es ist bei der vorliegenden Ausführung auch möglich, den Vakuumkolben 247 und den Pumpkolben 244 einteilig auszuführen.

An einer Mantelfläche des unteren Hohlzylinders 242, direkt unterhalb der unteren Fläche 245 des Pumpkolbens 244, ist eine Einmündung 254 von dem Anschluss 220 für den Monomer-Behälter 260 in den unteren Hohlzylinder 242 beziehungsweise den Hohlraum 241 vorgesehen. Die Einmündung 254 bildet ein Leitungsmittel für die Monomerflüssigkeit, so dass die Einmündung 254 als Teil einer Verbindungsleitung 238, 240 für die Monomerflüssigkeit aufgefasst werden kann, in der der untere Hohlzylinder 242 angeordnet ist.

In dem Anschluss 220 für den Monomer-Behälter 260 ist ein Sieb 256 oder Filter 256 angeordnet, mit dem Bruchstücke und Teile des geöffneten Monomer-Behälters 260 abgefangen werden können. Im Anschlussstutzen 218 ist unterhalb des Siebs 256 beziehungsweise des Filters 256 eine geneigte Bodenfläche 258 vorgesehen, die in Richtung der Einmündung 254 geneigt ist. Dadurch kann die gesamte Monomerflüssigkeit aus dem Monomer-Behälter 260 durch die Einmündung 254 in den unteren Hohlzylinder 242 fließen.

Der Monomer-Behälter 260 ist eine Glasampulle 260 mit einem abbrechbaren Ampullenkopf 262 und einem aufbrechbaren Genick, wobei das Genick den Ampullenkopf 262 mit dem Körper der Ampulle 260 verbindet. Durch die Elastizität der Aufnahme 221 für den Monomer-Behälter 260 und durch die Verdickung der Aufnahme 221 im Bereich des Genicks kann durch Biegen der Aufnahme 221 mit dem Monomer-Behälter 260 darin der Kopf 262 des Monomer-Behälters 260 abgebrochen werden. Die Aufnahme 221 mit der entsprechenden Form, insbesondere mit der Verdickung im Bereich des Genicks, bildet dadurch ein Öffnungsmittel 221 zum Öffnen des Monomer-Behälters 260. Andere Öffnungsmittel zum Abscheren des Kopfs 262 des Monomer-Behälters 260 sind ebenfalls umsetzbar.

Ein erfindungsgemäßes Verfahren kann mit der alternativen zweiten Vorrichtung nach den Figuren 7 und 8 beispielsweise wie folgt umgesetzt werden. Die Vorrichtung wird mit dem Standfuß 210 auf einem Tisch oder einer anderen passenden ebenen Unterlage aufgestellt. Der Monomer-Behälter 260 wird durch Knicken der elastischen Aufnahme 221 geöffnet, indem der Kopf 262 abgebrochen beziehungsweise aufgebrochen wird. Die Monomerflüssigkeit aus dem Monomer-Behälter 260 fließt durch das Sieb 256 beziehungsweise den Filter 256 wobei Bruchstücke des Monomer-Behälters 260 zurückgehalten werden. Durch die geneigte Bodenfläche 258 wird die Monomerflüssigkeit durch die Einmündung 254 in den unteren Hohlzylinder 242 beziehungsweise den Hohlraum 241 geleitet. Aufgrund der Belüftungsdurchgänge 223 kann Luft von außerhalb nachströmen, so dass die Monomerflüssigkeit zügig in den Hohlraum 241 fließen kann. Der freie Hohlraum 241 des unteren Hohlzylinders 242 wird vollständig mit der Monomerflüssigkeit gefüllt, da in dem Monomer-Behälter 260 mehr Monomerflüssigkeit enthalten ist, als der untere Hohlzylinder 242, beziehungsweise der Hohlraum 241 zusammen mit der Verbindungsleitung 238 bis zur Schlaufe 240 aufnehmen kann. Lufteinschlüsse entweichen durch die Einmündung 254, da diese am höchsten Punkt des durch den Hohlzylinder 242, den Boden 245 des Pumpkolbens 244 und die Verbindungsleitung 238 bis unterhalb der Schlaufe 240 begrenzten Raums angeordnet ist. Die Monomerflüssigkeit kann dabei nicht über den Scheitelpunkt der Schlaufe 240 fließen, da dieser deutlich oberhalb der der Einmündung 254 angeordnet ist und auch oberhalb des Flüssigkeitsspiegels der Monomerflüssigkeit im Anschluss 220 angeordnet ist, so dass die Monomerflüssigkeit daher nicht ohne Druck derart hoch steigen wird.

Anschließend wird über die Haube 215 und damit über die Stange 214 vom Anwender ein manueller Druck in Richtung des Standfußes 210 ausgeübt. Dadurch wird der Vakuumkolben 247 im Hohlraum 246 nach unten gedrückt und der Pumpkolben 244 wird in den Hohlzylinder 242 beziehungsweise den Hohlraum 241 eingedrückt. Der Pumpkolben 244 kann vollständig oder teilweise in den Hohlzylinder 242 beziehungsweise den Hohlraum 241 eingedrückt werden, um die gewünschte Menge Monomerflüssigkeit aus dem Hohlzylinder 242 durch die Verbindungsleitung 238 in den Innenraum 224 der Kartusche 201 zu überführen beziehungsweise zu pressen. Der Übertritt der Monomerflüssigkeit und die Standhöhe der Monomerflüssigkeit in dem Hohlraum 241 kann dabei über die Sichtfenster und die Skala im Gehäuse 239 kontrolliert werden. Die Menge der eingepressten Monomerflüssigkeit lässt sich durch unterschiedlich tiefes Einschieben des Pumpkolbens 244 in den Hohlzylinder 242 einstellen. Hierzu können Markierungen (nicht gezeigt) außen an der Haube 215 vorgesehen sein. Die Monomerflüssigkeit wird durch den Filter 232 in den Innenraum 224 der Kartusche 201 gepresst und steigt dort an und mischt sich mit dem Zementpulver, das in dem Innenraum 224 der Kartusche 201 gelagert ist.

Gleichzeitig wird durch die Bewegung des Vakuumkolbens 247 der Hohlraum 249 zwischen dem Vakuumkolben 247 und dem Abschlussdeckel 246 im oberen Hohlzylinder 212 vergrößert. Dadurch verringert sich der Gasdruck im Hohlraum 249. Der verringerte Druck wird mittels der Vakuumleitung 217 und den Vakuumanschluss des Dichtungskolbens 230 sowie durch den Porenfilter des Sterilisationskolbens 228 in den Innenraum 224 der Kartusche 201 geleitet. Dadurch wird der Innenraum 224 der Kartusche 201 mit der Bewegung des Vakuumkolbens 247 beziehungsweise der Kolben 244, 247 evakuiert. Da der Vakuumkolben 247 einen größeren Durchmesser hat als der Pumpkolben 244, wird weniger Volumen der Monomerflüssigkeit aus dem Hohlraum 241 in den Innenraum 224 der Kartusche 201 gedrückt, als Gas durch das sich im Hohlraum 249 vergrößernde Volumen aus dem Innenraum der Kartusche 201 evakuiert wird. Daher verringert sich der Druck im Innenraum der Kartusche 201 beim Eindrücken des Vakuumkolbens 247 und des Pumpkolbens 244.

Nachdem die gewünschte Menge Monomerflüssigkeit in den Innenraum 224 der Kartusche 201 eingefüllt wurde, wird durch Hineinschieben, Herausziehen und Drehen der Mischeinrichtung 226 beziehungsweise der Mischstange 204 mit Hilfe des Griffstücks 206 manuell die Monomerflüssigkeit und das Zementpulver gemischt und so der Zementteig beziehungsweise der PMMA-Knochenzement gemischt. Nach erfolgter Durchmischung wird die Mischstange 204 bis zum Anschlag aus dem Innenraum 224 der Kartusche 201 herausgezogen und abgebrochen, damit sie später nicht stört. Gegebenenfalls kann eine Arretierung des Austragskolbens 202 gelöst werden. Anschließend wird die Vakuumleitung 217 vom Vakuumanschluss des Dichtungskolbens 230 getrennt und die Kartusche 201 vom Standfuß 210 abgeschraubt. Ein Austragsrohr (nicht gezeigt) kann auf das Innengewinde am Kartuschenanschluss 236 geschraubt werden. Anschließend kann der fertig gemischte Knochenzement aus dem Innenraum 224 der Kartusche 201 durch die Austragsöffnung und das Austragsrohr durch Einpressen des Austragskolbens 202 ausgetrieben und appliziert werden.

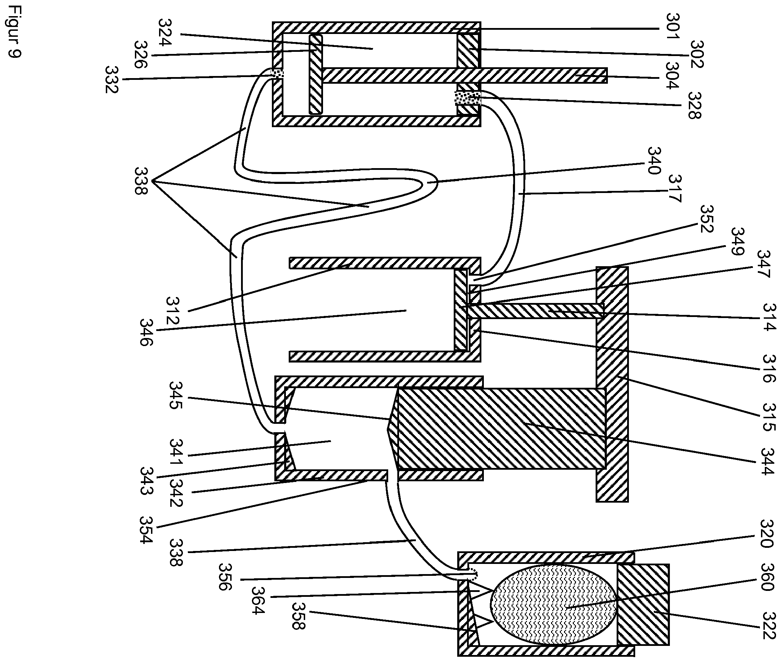

Figur 9 zeigt eine schematische Querschnittansicht einer alternativen vierten erfindungsgemäßen Vorrichtung als Full-Prepacked-Mischsystem, in der das schematische Funktionsprinzip dieser Vorrichtung dargestellt ist. Die Teile der vierten erfindungsgemäßen Vorrichtung sind selbstverständlich miteinander verbunden, teilweise lösbar miteinander verbunden. Der grundsätzliche Aufbau kann also weitgehend analog zu den vorherigen drei Ausführungsbeispielen nach den Figuren 1 bis 8 angenommen werden.

Die Vorrichtung umfasst eine Kartusche 301 (in Figur 9 links) mit einem Innenraum 324, in dem ein Zementpulver als erste Ausgangskomponente zur Herstellung des Knochenzements enthalten ist. Die Kartusche 301 ist auf der Oberseite durch einen Austragskolben 302 abgeschlossen, der axial in der Kartusche 301 beweglich ist. Eine Mischstange 304, die beweglich durch den Austragskolben 302 geführt ist, ist mit einer Mischeinrichtung 326 im Innenraum 324 der Kartusche 301 verbunden.

Die Vorrichtung umfasst ferner einen ersten Hohlzylinder 342 und einen zweiten Hohlzylinder 312, die im Unterschied zu den bisherigen Ausführungsbeispielen separat zueinander beziehungsweise nebeneinander angeordnet sind. In dem ersten Hohlzylinder 342 sind ein Hohlraum 341 und ein im Hohlraum 341 beweglicher Pumpkolben 344 vorgesehen. Der Pumpkolben 344 weist eine, zum trichterförmig geneigten Boden 343 des Hohlraums 341 passende untere Fläche 345 auf. Im Zentrum des trichterförmigen Bodens 343 ist ein Abfluss vorgesehen, an den eine Verbindungsleitung 338 angeschlossen ist, die den Hohlraum 341 für den Pumpkolben 344 mit dem Innenraum 324 der Kartusche 301 an deren Unterseite verbindet. In der Verbindung ist wieder ein Pulverundurchlässiger und Flüssigkeitsdurchlässiger Filter 332 vorgesehen. Analog zu den vorherigen Ausführungsbeispielen weist die Verbindungsleitung 338 eine Schlaufe 340 in passender Höhe auf.

Der zweite Hohlzylinder 312 ist zum Erzeugen eines Unterdrucks vorgesehen. Dazu ist in dem unten offenen Hohlraum 346 des Hohlzylinders 312 ein Vakuumkolben 347 axial beweglich angeordnet, der dicht mit den Innenwänden des Hohlzylinders 312 abschließt. Die Oberseite des Hohlzylinders 312 ist durch einen Abschlussdeckel 316 verschlossen. In dem Abschlussdeckel 316 ist eine druckdichte Durchführung für eine Stange 314 vorgesehen, wobei die Stange 314 mit dem Vakuumkolben 347 verbunden ist, so dass durch Einschieben der Stange 314 der Vakuumkolben 347 im Hohlraum 346 bewegt werden kann. Eine weitere Durchführung ist als Anschluss 352 für eine Vakuumleitung 317 vorgesehen. Die Vakuumleitung 317 verbindet den Zwischenraum 349 beziehungsweise den Hohlraum 349 zwischen dem Vakuumkolben 347 und dem Abschlussdeckel 316 mit dem Innenraum 324 der Kartusche 301. Dazu ist die Vakuumleitung 317 an eine Durchführung in dem Austragskolben 302 angeschlossen. In der Durchführung beziehungsweise in dem Austragskolben 302 ist eine Porenscheibe 328 vorgesehen.

Im Unterschied zu den bisherigen Ausführungsbeispielen sind bei der vorliegenden Vorrichtung der Vakuumkolben 347 und der Pumpkolben 344 nicht direkt miteinander verbunden. Stattdessen ist ein gemeinsames Bedienelement 315 vorgesehen, mit dem die Stange 314 und damit der Vakuumkolben 347 sowie der Pumpkolben 344 nach unten gedrückt werden können (in Figur 9 nach unten).

Über eine Einmündung 354 ist eine Verbindungsleitung 238 angeschlossen, die bodenseitig mit einem Anschluss 320 für einen Monomer-Behälter 360 verbunden ist. In dem Monomer-Behälter 360 ist vorliegend ein metallbeschichteter Folienbeutel 360, der im Inneren des Anschlusses 320 mit Dornen 364 oder Klingen aufgeschlitzt werden kann. Dazu kann der Monomer-Behälter 360 mit einem Deckel 322 (beispielsweise einem Schraubdeckel) auf die Dorne 364 gedrückt werden. Der Boden 358 des Anschlusses 320 ist geneigt, damit die Monomerflüssigkeit gut aus dem Anschluss 320 ausfließen kann. Belüftungsdurchführungen (nicht gezeigt) können in dem Deckel 322 vorgesehen sein, damit Luft bei Ausfließen der Monomerflüssigkeit in den Anschluss 320 nachströmen kann. Um Fetzen oder Partikel des aufgeschlitzten oder aufgestochenen Monomer-Behälters 360 zurückhalten zu können, ist im Anschluss 320 ein Sieb 356 vorgesehen.

Die Monomerflüssigkeit fließt aus dem Monomer-Behälter 360 durch die Verbindungsleitung 338 in den Hohlraum 341. Ein Übersteigen des Flüssigkeitsspiegels der Monomerflüssigkeit über die Schlaufe 340 ist nicht möglich, da diese höher als die Einmündung 354 angeordnet ist. Mit dem Bedienelement 315 wird ein Druck auf den Pumpkolben 344 ausgeübt. Mit dem Pumpkolben 344 wird die Monomerflüssigkeit aus dem Hohlraum 341 in den Innenraum 324 der Kartusche 301 gedrückt. Gleichzeitig drückt das Bedienelement 315 den Vakuumkolben 347 über die Stange 314 nach unten. Dabei wird der Hohlraum 349 vergrößert und so Luft aus dem Innenraum 324 der Kartusche 301 über die Vakuumleitung 317 in den Hohlraum 349 gezogen. Die Monomerflüssigkeit kann mit dem Zementpulver in dem Innenraum 324 der Kartusche 301 mit der Mischeinrichtung 326 gemischt werden. Anschließend wird die Kartusche 301 von der Verbindungsleitung 338 und der Vakuumleitung 317 getrennt. Hierzu kann der Durchgang durch den Austragskolben 302 verschlossen werden. Mit dem Austragskolben 302 kann dann das fertige Gemisch durch die Öffnung im Boden der Kartusche 301, aus der der Filter 332 entfernt wurde, ausgetrieben werden. Dabei kann ein Austragsrohr (nicht gezeigt) an der Öffnung im Boden der Kartusche 301 (in Figur 9 unten) befestigt werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101, 201, 301: Kartusche
- 2, 102, 202, 302: Austragskolben
- 4, 104, 204, 304: Mischstange
- 6, 106, 206: Griffstück
- 8, 108, 208: Stutzen
- 10, 110, 210: Standfuß
- 12, 112, 212, 312: Hohlzylinder für den Vakuumkolben
- 14, 114, 214, 314: Stange
- 15: Drucktaste
- 16, 116, 216, 316: Abschlussdeckel
- 17, 117, 217, 317: Vakuumleitung
- 18, 118, 218: Anschlussstutzen
- 20, 120, 220, 320: Anschluss für den Monomer-Behälter
- 21, 121, 221: Elastische Aufnahme für den Monomer-Behälter / Öffnungsmittel
- 22, 122, 222, 322: Deckel
- 24, 124, 224, 324: Innenraum der Kartusche
- 26, 126, 226, 326: Mischflügel / Mischeinrichtung
- 28, 128, 228: Sterilisationskolben
- 29, 129, 229: Dichtung
- 30, 130, 230: Dichtungskolben
- 32, 132, 232, 332: Pulverundurchlässiger und Flüssigkeitsdurchlässiger Filter
- 34, 134, 234: Standfuß-Anschluss mit Außengewinde
- 36, 136, 236: Kartuschenanschluss mit Innengewinde
- 38, 138, 238, 338: Verbindungsleitung
- 40, 140, 240, 340: Schlaufe der Verbindungsleitung
- 41, 141, 241, 341: Hohlraum für den Pumpkolben
- 42, 142, 242, 342: Hohlzylinder für den Pumpkolben
- 43, 143, 243, 343: Boden des Hohlzylinders für den Pumpkolben
- 44, 144, 244, 344: Pumpkolben
- 45, 145, 245, 345: Untere Fläche des Pumpkolbens
- 46, 146, 246, 346: Offener (unterer) Hohlraum für den Vakuumkolben
- 47, 147, 247, 347: Vakuumkolben
- 48, 148, 248: Dichtung
- 49, 149, 249, 349: Geschlossener (oberer) Hohlraum für den Vakuumkolben
- 50, 150, 250: Öffnung
- 52, 152, 252, 352: Anschluss für Vakuumleitung
- 53, 153, 253: Dichtung
- 54, 154, 254, 354: Einmündung in den Hohlzylinder für den Pumpkolben
- 56, 156, 256, 356: Sieb / Filter
- 58, 158, 258, 358: Geneigte Bodenfläche des Anschlusses für Monomer-Behälter
- 60, 160, 260: Glasampulle / Monomer-Behälter
- 62, 162, 262: Kopf der Glasampulle / Kopf des Monomer-Behälters
- 115: Sicherung
- 151: Gespannte Druckfeder
- 215: Haube
- 223: Belüftungsdurchgang
- 239: Gehäuse für Schlaufe der Verbindungsleitung
- 315: Gemeinsames Bedienelement
- 328: Verschließbarer Porenfilter
- 360: Monomer-Beutel / Monomer-Behälter
- 364: Dorn

## Patentansprüche

1. Vorrichtung zum Mischen von Polymethylmethacrylat-Knochenzement und zum Lagern einer Monomerflüssigkeit und eines Zementpulvers als Ausgangskomponenten des Knochenzements, die Vorrichtung aufweisend eine Kartusche (1, 101, 201, 301) mit einem Innenraum (24, 124, 224, 324) zum Mischen des Knochenzements, der auf einer Seite durch einen beweglichen Austragskolben (2, 102, 202, 302) verschlossen ist,
einen Monomer-Behälter (60, 160, 260, 360) für eine Monomerflüssigkeit und/oder einen Anschluss (20, 120, 220, 320) zur Befestigung eines Monomer-Behälters (60, 160, 260, 360) für eine Monomerflüssigkeit, so dass der Monomer-Behälter (60, 160, 260, 360) in der Vorrichtung derart zu öffnen ist, dass die Monomerflüssigkeit aus dem Monomer-Behälter (60, 160, 260, 360) in die Vorrichtung ausläuft,
eine Verbindungsleitung (38, 138, 238, 338), durch die die Monomerflüssigkeit in den Innenraum (24, 124, 224, 324) der Kartusche (1, 101, 201, 301) zu leiten ist, wobei
ein erster Hohlzylinder (42, 142, 242, 342) mit der Verbindungsleitung (38, 138, 238, 338) verbunden ist und ein zweiter Hohlzylinder (12, 112, 212, 312) über eine Vakuumleitung (17, 117, 217, 317) mit dem Innenraum (24, 124, 224, 324) der Kartusche (1, 101, 201, 301) verbunden ist, wobei in dem ersten Hohlzylinder (42, 142, 242, 342) ein axial im ersten Hohlzylinder (42, 142, 242, 342) verschiebbarer Pumpkolben (44, 144, 244, 344) angeordnet ist und in dem zweiten Hohlzylinder (12, 112, 212, 312) ein axial im zweiten Hohlzylinder (12, 112, 212, 312) verschiebbarer Vakuumkolben (47, 147, 247, 347) angeordnet ist, wobei der Pumpkolben (44, 144, 244, 344) und der Vakuumkolben (47, 147, 247, 347) zeitgleich bewegbar sind, **dadurch gekennzeichnet, dass**
der erste Hohlzylinder (42, 142, 242, 342) derart mit dem Monomer-Behälter (60, 160, 260, 360) und/oder dem Anschluss (20, 120, 220, 320) zur Befestigung eines Monomer-Behälters (60, 160, 260, 360) verbunden ist, dass die Monomerflüssigkeit aus dem geöffneten Monomer-Behälter (60, 160, 260, 360) oder aus einem geöffneten angeschlossenen Monomer-Behälter (60, 160, 260, 360) in den ersten Hohlzylinder (42, 142, 242, 342) fließt und die Verbindungsleitung (38, 138, 238, 338) den ersten Hohlzylinder (42, 142, 242, 342) mit dem Innenraum (24, 124, 224, 324) der Kartusche (1, 101, 201, 301) derart verbindet, dass mit dem Pumpkolben (44, 144, 244, 344) Monomerflüssigkeit aus dem ersten Hohlzylinder (42, 142, 242, 342) durch Betätigen des Pumpkolbens (44, 144, 244, 344) durch die Verbindungsleitung (38, 138, 238, 338) in den Innenraum (24, 124, 224, 324) der Kartusche (1, 101, 201, 301) gedrückt werden kann.

2. Vorrichtung nach Anspruch 1, **dadurch gekennzeichnet, dass**
der Pumpkolben (44, 144, 244, 344) und der Vakuumkolben (47, 147, 247, 347) derart miteinander verbunden sind oder derart über ein gemeinsames Bedienelement (315) bewegt werden, dass sich beim Einschieben des Pumpkolbens (44, 144, 244, 344) in den ersten Hohlzylinder (42, 142, 242, 342) der Vakuumkolben (47, 147, 247, 347) im zweiten Hohlzylinder (12, 112, 212, 312) von der Verbindung (52, 152, 252, 352) zur Vakuumleitung (17, 117, 217, 317) weg bewegt, wobei vorzugsweise sich dabei das Volumen zwischen dem Vakuumkolben (47, 147, 247, 347) und der Verbindung (52, 152, 252, 352) zur Vakuumleitung (17, 117, 217, 317) im zweiten Hohlzylinder (12, 112, 212, 312) vergrößert, so dass durch den im zweiten Hohlzylinder (12, 112, 212, 312) entstehenden Unterdruck ein Gas aus dem Innenraum (24, 124, 224, 324) der Kartusche (1, 101, 201, 301) durch die Vakuumleitung (17, 117, 217, 317) in den zweiten Hohlzylinder (12, 112, 212, 312) strömt.

3. Vorrichtung nach einem der vorangehenden Ansprüche, **dadurch gekennzeichnet, dass**
der erste Hohlzylinder (42, 142, 242, 342) zwischen dem Monomer-Behälter (60, 160, 260, 360) oder dem Anschluss (20, 120, 220, 320) für den Monomer-Behälter (60, 160, 260, 360) und dem Innenraum (24, 124, 224, 324) der Kartusche (1, 101, 201, 301) angeordnet ist, vorzugsweise in der Verbindungsleitung (38, 138, 238, 338) zwischen dem Monomer-Behälter (60, 160, 260, 360) oder dem Anschluss (20, 120, 220, 320) für den Monomer-Behälter (60, 160, 260, 360) und dem Innenraum (24, 124, 224, 324) der Kartusche (1, 101, 201, 301) angeordnet ist.

4. Vorrichtung nach einem der vorangehenden Ansprüche, **dadurch gekennzeichnet, dass**
der Pumpkolben (44, 144, 244, 344) und der erste Hohlzylinder (42, 142, 242, 342) eine geringere Querschnittsfläche aufweisen als der Vakuumkolben (47, 147, 247, 347) und der zweite Hohlzylinder (12, 112, 212, 312), vorzugsweise der Vakuumkolben (47, 147, 247, 347) und der zweite Hohlzylinder (12, 112, 212, 312) eine mindestens doppelt so große Querschnittsfläche aufweisen wie der Pumpkolben (44, 144, 244, 344) und der erste Hohlzylinder (42, 142, 242, 342).

5. Vorrichtung nach einem der vorangehenden Ansprüche, **dadurch gekennzeichnet, dass**
der Pumpkolben (44, 144, 244, 344) und der Vakuumkolben (47, 147, 247, 347) miteinander verbunden sind, bevorzugt fest miteinander verbunden sind, besonders bevorzugt einteilig aufgebaut sind.

6. Vorrichtung nach Anspruch 5, **dadurch gekennzeichnet, dass**
die der Vakuumleitung (17, 117, 217) zugewandte Seite des Vakuumkolbens (47, 147, 247) auf der Rückseite des Pumpkolbens (44, 144, 244) angeordnet ist und dementsprechend die Verbindungsleitung (38, 138, 238) zugewandte Seite des Pumpkolbens (44, 144, 244) auf der Rückseite des Vakuumkolbens (47, 147, 247) angeordnet ist.

7. Vorrichtung nach einem der vorangehenden Ansprüche, **dadurch gekennzeichnet, dass**
die Vakuumleitung (17, 117, 217, 317) durch den Austragskolben (2, 102, 202, 302) mit dem Innenraum (24, 124, 224, 324) der Kartusche (1, 101, 201, 301) verbunden ist, wobei vorzugsweise im Austragskolben (2, 102, 202, 302) ein Filter (328) oder ein Sieb angeordnet ist über den oder das die Vakuumleitung (17, 117, 217, 317) mit dem Innenraum (24, 124, 224, 324) der Kartusche (1, 101, 201, 301) verbunden ist.

8. Vorrichtung nach einem der vorangehenden Ansprüche, **dadurch gekennzeichnet, dass**
der Monomer-Behälter (60, 160, 260, 360) in einem flexiblen Ampullen-Behälter (21, 121, 221) angeordnet ist oder anzuordnen ist, wobei vorzugsweise in dem Ampullen-Behälter (21, 121, 221) zumindest eine Belüftungsöffnung (223) vorgesehen ist.

9. Vorrichtung nach einem der vorangehenden Ansprüche, **dadurch gekennzeichnet, dass**
in der Kartusche (1, 101, 201, 301) eine Mischeinrichtung (26, 126, 226, 326) angeordnet ist, die von außen bedienbar ist, wobei vorzugsweise die Mischeinrichtung (26, 126, 226, 326) über eine Mischstange (4, 104, 204, 304) bedienbar ist, die durch eine Durchführung in dem Austragskolben (2, 102, 202, 302) ins Innere der Kartusche (1, 101, 201, 301) geführt und beweglich gelagert ist.

10. Vorrichtung nach einem der vorangehenden Ansprüche, **dadurch gekennzeichnet, dass**
das Zementpulver im Innenraum (24, 124, 224, 324) der Kartusche (1, 101, 201, 301) enthalten ist.

11. Vorrichtung nach einem der vorangehenden Ansprüche, **dadurch gekennzeichnet, dass**
zwischen der Verbindungsleitung (38, 138, 238, 338) und dem Innenraum (24, 124, 224, 324) der Kartusche (1, 101, 201, 301) ein für das Zementpulver undurchlässiger und für die Monomerflüssigkeit durchlässiger Filter (32, 132, 232, 332) angeordnet ist.

12. Vorrichtung nach einem der vorangehenden Ansprüche, **dadurch gekennzeichnet, dass**
die Vorrichtung einen Standfuß (10, 110, 210) aufweist, in dem zumindest ein Teil der Verbindungsleitung (38, 138, 238, 338) angeordnet ist, wobei die Kartusche (1, 101, 201, 301) lösbar mit dem Standfuß (10, 110, 210) verbunden ist, insbesondere über ein Schraubgewinde lösbar mit dem Standfuß (10, 110, 210) verbunden ist, wobei bevorzugt gegebenenfalls der für das Zementpulver undurchlässige und für die Monomerflüssigkeit durchlässige Filter (32, 132, 232, 332) in dem Standfuß (10, 110, 210) der Vorrichtung angeordnet ist, besonders bevorzugt in der Verbindung (34, 134, 234) zur Kartusche (1, 101, 201, 301) des Standfußes (10, 110, 210) angeordnet ist.

13. Vorrichtung nach Anspruch 12, **dadurch gekennzeichnet, dass**
der erste Hohlzylinder (42, 142, 242, 342), der zweite Hohlzylinder (12, 112, 212, 312) und der Monomer-Behälter (60, 160, 260, 360) oder der erste Hohlzylinder (42, 142, 242, 342), der zweite Hohlzylinder (12, 112, 212, 312) und der Anschluss (20, 120, 220, 320) zur Befestigung des Monomer-Behälters (60, 160, 260, 360) mit dem Standfuß (10, 110, 210) verbunden sind, bevorzugt unlösbar mit dem Standfuß (10, 110, 210) verbunden sind.

14. Vorrichtung nach einem der vorangehenden Ansprüche, **dadurch gekennzeichnet, dass**
der Monomer-Behälter (60, 160, 260, 360) für die Monomerflüssigkeit oder der Anschluss (20, 120, 220, 320) zur Befestigung des Monomer-Behälters (60, 160, 260, 360) an einer Mantelfläche des ersten Hohlzylinders (42, 142, 242, 342) in den ersten Hohlzylinder (42, 142, 242, 342) mündet, bevorzugt unmittelbar unterhalb des Pumpkolbens (44, 144, 244, 344) in den ersten Hohlzylinder (42, 142, 242, 342) mündet.

15. Vorrichtung nach einem der vorangehenden Ansprüche, **dadurch gekennzeichnet, dass**
der Monomer-Behälter (60, 160, 260, 360) oberhalb der der Verbindung (54, 154, 254, 354) zum ersten Hohlzylinder (42, 142, 242, 342) angeordnet ist.

16. Vorrichtung nach einem der vorangehenden Ansprüche, **dadurch gekennzeichnet, dass**
der Pumpkolben (44, 144, 244, 344) manuell axial im ersten Hohlzylinder (42, 142, 242, 342) bewegt werden kann, bevorzugt manuell axial in den ersten Hohlzylinder (42, 142, 242, 342) eingepresst werden kann, und/oder der Vakuumkolben (47, 147, 247, 347) manuell axial im zweiten Hohlzylinder (12, 112, 212, 312) bewegt werden kann.

17. Vorrichtung nach einem der vorangehenden Ansprüche, **dadurch gekennzeichnet, dass**
der erste Hohlzylinder (42, 142, 242, 342) und/oder der zweite Hohlzylinder (12, 112, 212, 312) ein Innengewinde aufweist und der Pumpkolben (44, 144, 244, 344) und/oder der Vakuumkolben (47, 147, 247, 347) ein dazu passendes Außengewinde aufweist, so dass der Pumpkolben (44, 144, 244, 344) und/oder der Vakuumkolben (47, 147, 247, 347) in den ersten Hohlzylinder (42, 142, 242, 342) und/oder zweiten Hohlzylinder (12, 112, 212, 312) einschraubbar ist, um die Monomerflüssigkeit aus dem ersten Hohlzylinder (42, 142, 242, 342) in den Innenraum (24, 124, 224, 324) der Kartusche (1, 101, 201, 301) zu pressen und/oder um Luft aus dem Innenraum (24, 124, 224, 324) der Kartusche (1, 101, 201, 301) in den zweiten Hohlzylinder (12, 112, 212, 312) zu ziehen.

18. Vorrichtung nach einem der Ansprüche 1 bis 16, **dadurch gekennzeichnet, dass**
die Vorrichtung zumindest eine gespannte Druckfeder (151) und zumindest eine Arretierung aufweist, wobei die Druckfeder (151), der Vakuumkolben (47, 147, 247, 347) und/oder der Pumpkolben (44, 144, 244, 344) mit der zumindest einen Arretierung lösbar arretiert ist oder sind, wobei bei gelöster Arretierung die zumindest eine Druckfeder (151) einen Druck auf den Pumpkolben (44, 144, 244, 344) und/oder den Vakuumkolben (47, 147, 247, 347) ausübt, so dass der Pumpkolben (44, 144, 244, 344) in den ersten Hohlzylinder (42, 142, 242, 342) gepresst wird und/oder der Vakuumkolben (47, 147, 247, 347) von dem Anschluss (52, 152, 252, 352) zur Vakuumleitung (17, 117, 217, 317) weg im zweiten Hohlzylinder (12, 112, 212, 312) gedrückt wird.

19. Vorrichtung nach einem der vorangehenden Ansprüche, **dadurch gekennzeichnet, dass**
die Verbindungsleitung (38, 138, 238, 338) zwischen dem ersten Hohlzylinder (42, 142, 242, 342) und dem Innenraum (24, 124, 224, 324) der Kartusche (1, 101, 201, 301) eine nach oben weisende Schlaufe (40, 140, 240, 340) aufweist, wobei der höchste Punkt der Schlaufe (40, 140, 240, 340) oberhalb einer Einmündung (54, 154, 254, 354) des Monomer-Behälters (60, 160, 260, 360) oder des Anschlusses (20, 120, 220, 320) für den Monomer-Behälter (60, 160, 260, 360) in den ersten Hohlzylinder (42, 142, 242, 342) liegt.

20. Verfahren zum Mischen eines Knochenzements, insbesondere mit einer Vorrichtung nach einem der Ansprüche 1 bis 19, mit den chronologischen Schritten
A) ein Monomer-Behälter (60, 160, 260, 360) wird geöffnet,
B) eine Monomerflüssigkeit fließt aus dem Monomer-Behälter (60, 160, 260, 360) in einen ersten Hohlzylinder (42, 142, 242, 342), wobei der erste Hohlzylinder (42, 142, 242, 342) auf einer Seite durch einen Pumpkolben (44, 144, 244, 344) begrenzt ist,
C) der Pumpkolben (44, 144, 244, 344) wird in den ersten Hohlzylinder (42, 142, 242, 342) eingedrückt und die Monomerflüssigkeit dadurch aus dem ersten Hohlzylinder (42, 142, 242, 342) und durch eine Verbindungsleitung (38, 138, 238, 338) in den Innenraum (24, 124, 224, 324) einer Kartusche (1, 101, 201, 301) eingepresst, wobei sich in dem Innenraum (24, 124, 224, 324) der Kartusche (1, 101, 201, 301) ein Zementpulver befindet,
D) mit der Bewegung des Pumpkolbens (44, 144, 244, 344) wird ein mit dem Pumpkolben (44, 144, 244, 344) verbundener oder parallel angetriebener Vakuumkolben (47, 147, 247, 347) in einem zweiten Hohlzylinder (12, 112, 212, 312) von einem Anschluss (52, 152, 252, 352) einer Vakuumleitung (17, 117, 217, 317) weg bewegt, wobei durch die Bewegung des Vakuumkolbens (47, 147, 247, 347) der Gasdruck zwischen dem Anschluss (52, 152, 252, 352) der Vakuumleitung (17, 117, 217, 317) und dem Vakuumkolben (47, 147, 247, 347) im zweiten Hohlzylinder (12, 112, 212, 312) verringert wird und Gas durch die Vakuumleitung (17, 117, 217, 317) aus dem an die Vakuumleitung (17, 117, 217, 317) angeschlossenen Innenraum (24, 124, 224, 324) der Kartusche (1, 101, 201, 301) evakuiert wird, und
E) die Monomerflüssigkeit und das Zementpulver werden in dem Innenraum (24, 124, 224, 324) der Kartusche (1, 101, 201, 301) gemischt.

21. Verfahren nach Anspruch 20, **dadurch gekennzeichnet, dass**
die Luft aus dem Innenraum (24, 124, 224, 324) der Kartusche (1, 101, 201, 301) auf der der Einmündung der Verbindungsleitung (38, 138, 238, 338) gegenüberliegenden Seite durch die Vakuumleitung (17, 117, 217, 317) abgesaugt wird, wobei vorzugsweise die Verbindungsleitung (38, 138, 238, 338) an der Unterseite des Innenraums (24, 124, 224, 324) in den Innenraum (24, 124, 224, 324) der Kartusche (1, 101, 201, 301) mündet und die Vakuumleitung (17, 117, 217, 317) an der Oberseite des Innenraums (24, 124, 224, 324) in den Innenraum (24, 124, 224, 324) der Kartusche (1, 101, 201, 301) mündet.

22. Verfahren nach Anspruch 20 oder 21, **dadurch gekennzeichnet, dass**
die Monomerflüssigkeit und das Zementpulver in dem Innenraum (24, 124, 224, 324) mit einer Mischeinrichtung (26, 126, 226, 326) gemischt werden, indem die Mischeinrichtung (26, 126, 226, 326) durch Bewegen einer drehbar und in Längsrichtung verschiebbar in den Innenraum (24, 124, 224, 324) der Kartusche (1, 101, 201, 301) geführten Mischstange (4, 104, 204, 304) bedient wird, wobei bevorzugt nach dem Mischen die Mischstange (4, 104, 204, 304) bis zum Anschlag aus dem Innenraum (24, 124, 224, 324) der Kartusche (1, 101, 201, 301) herausgezogen wird und besonders bevorzugt die Mischstange (4, 104, 204, 304) nach dem Herausziehen bis zum Anschlag an einer Sollbruchstelle abgebrochen wird.

23. Verfahren nach einem der Ansprüche 20 bis 22, **dadurch gekennzeichnet, dass**
der Pumpkolben (44, 144, 244, 344) mit einem gespannten elastischen Federelement (151) in den ersten Hohlzylinder (42, 142, 242, 342) eingedrückt wird und/oder der Vakuumkolben (47, 147, 247, 347) mit einem gespannten elastischen Federelement (151) im zweiten Hohlzylinder (12, 112, 212, 312) bewegt wird, wobei hierfür vorzugsweise zuvor zumindest eine Arretierung gelöst wird, die in den Pumpkolben (44, 144, 244, 344), den Vakuumkolben (47, 147, 247, 347) und/oder das Federelement (151) greift oder greifen.

24. Verfahren nach einem der Ansprüche 20 bis 23, **dadurch gekennzeichnet, dass**
die Kartusche (1, 101, 201, 301) mit dem fertig gemischten Zementteig von der Verbindungsleitung (38, 138, 238, 338), der Vakuumleitung (17, 117, 217, 317), dem ersten Hohlzylinder (42, 142, 242, 342), dem zweiten Hohlzylinder (12, 112, 212, 312) und dem Monomer-Behälter (60, 160, 260, 360) gelöst wird und der fertig gemischte Zementteig durch Vortreiben eines Austragskolbens (2, 102, 202, 302), der axial beweglich in der Kartusche (1, 101, 201, 301) gelagert ist und der den Innenraum (24, 124, 224, 324) der Kartusche (1, 101, 201, 301) auf einer Seite begrenzt, aus dem Innenraum (24, 124, 224, 324) der Kartusche (1, 101, 201, 301) ausgetragen wird.

## Claims

1. Device for the mixing of polymethylmethacrylate bone cement and for storage of a monomer liquid and a cement powder as starting components of the bone cement, the device comprising
a cartridge (1, 101, 201, 301) with an internal space (24, 124, 224, 324) for mixing the bone cement that is closed on one side by means of a mobile dispensing plunger (2, 102, 202, 302);
a monomer container (60, 160, 260, 360) for a monomer liquid and/or a connector (20, 120, 220, 320) for attachment of a monomer container (60, 160, 260, 360) for a monomer liquid such that the monomer container (60, 160, 260, 360) is openable appropriately in the device such that the monomer liquid flows from the monomer container (60, 160, 260, 360) into the device;
a connecting conduit (38, 138, 238, 338) through which the monomer liquid can be guided into the internal space (24, 124, 224, 324) of the cartridge (1, 101, 201, 301), whereby
a first hollow cylinder (42, 142, 242, 342) is connected to the connecting conduit (38, 138, 238, 338) and a second hollow cylinder (12, 112, 212, 312) is connected, via a vacuum conduit (17, 117, 217, 317), to the internal space (24, 124, 224, 324) of the cartridge (1, 101, 201, 301), whereby a pumping plunger (44, 144, 244, 344) that can be shifted axially in the first hollow cylinder (42, 142, 242, 342) is arranged in the first hollow cylinder (42, 142, 242, 342) and a vacuum plunger (47, 147, 247, 347) that can be shifted axially in the second hollow cylinder (12, 112, 212, 312) is arranged in the second hollow cylinder (12, 112, 212, 312), whereby the pumping plunger (44, 144, 244, 344) and the vacuum plunger can be moved simultaneously (47, 147, 247, 347),
**characterized in that**
the first hollow cylinder (42, 142, 242, 342) is connected to the monomer container (60, 160, 260, 360) and/or the connector (20, 120, 220, 320) for attachment of a monomer container (60, 160, 260, 360) in appropriate manner such that the monomer liquid flows from the opened monomer container (60, 160, 260, 360) or an opened attached monomer container (60, 160, 260, 360) into the first hollow cylinder (42, 142, 242, 342) and the connecting conduit (38, 138, 238, 338) connects the first hollow cylinder (42, 142, 242, 342) to the internal space (24, 124, 224, 324) of the cartridge (1, 101, 201, 301) in appropriate manner such that the pumping plunger (44, 144, 244, 344) can be used to push monomer liquid from the first hollow cylinder (42, 142, 242, 342) through the connecting conduit (38, 138, 238, 338) into the internal space (24, 124, 224, 324) of the cartridge (1, 101, 201, 301) by actuating the pumping plunger (44, 144, 244, 344).

2. Device according to claim 1, **characterised in that**
the pumping plunger (44, 144, 244, 344) and the vacuum plunger (47, 147, 247, 347) are connected to each other appropriately or are moved by means of a shared operating element (315) such that, when the pumping plunger (44, 144, 244, 344) is being slid into the first hollow cylinder (42, 142, 242, 342), the vacuum plunger (47, 147, 247, 347) in the second hollow cylinder (12, 112, 212, 312) moves away from the connection to the vacuum conduit (17, 117, 217, 317), whereby the volume between the vacuum plunger (47, 147, 247, 347) and the connection 52, 152, 252, 352) to the vacuum conduit (17, 117, 217, 317) in the second hollow cylinder (12, 112, 212, 312) preferably enlarges such that the negative pressure arising in the second hollow cylinder (12, 112, 212, 312) causes a gas to flow from the internal space (24, 124, 224, 324) of the cartridge (1, 101, 201, 301) through the vacuum conduit (17, 117, 217, 317) into the second hollow cylinder (12, 112, 212, 312).

3. Device according to any one of the preceding claims, **characterised in that**
the first hollow cylinder (42, 142, 242, 342) is arranged between the monomer container (60, 160, 260, 360) or the connector (20, 120, 220, 320) for the monomer container (60, 160, 260, 360) and the internal wall (24, 124, 224, 324) of the cartridge (1, 101, 201, 301), preferably is arranged in the connecting conduit (38, 138, 238, 338) between the monomer container (60, 160, 260, 360) or the connector (20, 120, 220, 320) for the monomer container (60, 160, 260, 360) and the internal wall (24, 124, 224, 324) of the cartridge (1, 101, 201, 301).

4. Device according to any one of the preceding claims, **characterised in that**
the pumping plunger (44, 144, 244, 344) and the first hollow cylinder (42, 142, 242, 342) comprise a smaller cross-sectional area than the vacuum plunger (47, 147, 247, 347) and the second hollow cylinder (12, 112, 212, 312), preferably the vacuum plunger (47, 147, 247, 347) and the second hollow cylinder (12, 112, 212, 312) comprise a cross-sectional area at least twice as large as the pumping plunger (44, 144, 244, 344) and the first hollow cylinder (42, 142, 242, 342).

5. Device according to any one of the preceding claims, **characterised in that**
the pumping plunger (44, 144, 244, 344) and the vacuum plunger (47, 147, 247, 347) are connected to each other, preferably are affixed to each other, particularly preferably are designed as a single part.

6. Device according to claim 5, **characterised in that**
the side of the vacuum plunger (47, 147, 247) facing the vacuum conduit (17, 117, 217) is arranged on the rear side of the pumping plunger (44, 144, 244) and, correspondingly, the side of the pumping plunger (44, 144, 244) facing the connecting conduit (38, 138, 238) is arranged on the rear side of the vacuum plunger (47, 147, 247).

7. Device according to any one of the preceding claims, **characterised in that**
the vacuum conduit (17, 117, 217, 317) is connected to the internal space (24, 124, 224, 324) of the cartridge (1, 101, 201, 301) by means of the dispensing plunger (2, 102, 202, 302), whereby the dispensing plunger (2, 102, 202, 302) preferably has a filter (328) or a screen arranged in it by means of which the vacuum conduit (17, 117, 217, 317) is connected to the internal space (24, 124, 224, 324) of the cartridge (1, 101, 201, 301).

8. Device according to any one of the preceding claims, **characterised in that**
the monomer container (60, 160, 260, 360) is arranged or arrangeable in a flexible ampoule container (21, 121, 221), whereby it is preferable to provide at least one ventilation opening (223) in the ampoule container (21, 121, 221).

9. Device according to any one of the preceding claims, **characterised in that**
a mixing facility (26, 126, 226, 326) that can be operated from outside is arranged in the cartridge (1, 101, 201, 301), whereby the mixing facility (26, 126, 226, 326) preferably can be operated by means of a mixing rod (4, 104, 204, 304) that is guided through a feed-through in the dispensing plunger (2, 102, 202, 302) into the interior of the cartridge (1, 101, 201, 301) and is supported such as to be mobile.

10. Device according to any one of the preceding claims, **characterised in that**
the internal space (24, 124, 224, 324) of the cartridge (1, 101, 201, 301) contains the cement powder.

11. Device according to any one of the preceding claims, **characterised in that**
a filter (32, 132, 232, 332) that is impermeable for the cement powder and permeable for the monomer liquid is arranged between the connecting conduit (38, 138, 238, 338) and the internal space (24, 124, 224, 324) of the cartridge (1, 101, 201, 301).

12. Device according to any one of the preceding claims, **characterised in that**
the device comprises a base (10, 110, 210), in which at least a part of the connecting conduit (38, 138, 238, 338) is arranged, whereby the cartridge (1, 101, 201, 301) is connected to the base (10, 110, 210) in detachable manner, in particular is connected to the base (10, 110, 210) in detachable manner by means of a screw thread, whereby the filter (32, 132, 232, 332), if any, that is impermeable for the cement powder and permeable for the monomer liquid is arranged in the base (10, 110, 210) of the device, particularly preferably is arranged in the connection to the cartridge (1, 101, 201, 301) of the base (10, 110, 210).

13. Device according to claim 12, **characterised in that**
the first hollow cylinder (42, 142, 242, 342), the second hollow cylinder (12, 112, 212, 312), and the monomer container (60, 160, 260, 360) or the first hollow cylinder (42, 142, 242, 342), the second hollow cylinder (12, 112, 212, 312), and the connector (20, 120, 220, 320) for attaching the monomer container (60, 160, 260, 360) are connected to the base (10, 110, 210), preferably are connected to the base (10, 110, 210) in non-detachable manner.

14. Device according to any one of the preceding claims, **characterised in that**
the monomer container (60, 160, 260, 360) for the monomer liquid or the connector (20, 120, 220, 320) for attaching the monomer container (60, 160, 260, 360) merge into the first hollow cylinder (42, 142, 242, 342) on a jacket surface of the first hollow cylinder (42, 142, 242, 342), preferably merge into the first hollow cylinder (42, 142, 242, 342) right below the pumping plunger (44, 144, 244, 344).

15. Device according to any one of the preceding claims, **characterised in that**
the monomer container (60, 160, 260, 360) is arranged above the connection (54, 154, 254, 354) to the first hollow cylinder (42, 142, 242, 342).

16. Device according to any one of the preceding claims, **characterised in that**
the pumping plunger (44, 144, 244, 344) can be moved axially in the first hollow cylinder (42, 142, 242, 342) by hand, preferably can be pressed axially into the first hollow cylinder (42, 142, 242, 342) by hand and/or the vacuum plunger (47, 147, 247, 347) can be moved axially in the second hollow cylinder (12, 112, 212, 312) by hand.

17. Device according to any one of the preceding claims, **characterised in that**
the first hollow cylinder (42, 142, 242, 342) and/or the second hollow cylinder (12, 112, 212, 312) comprise an internal thread and the pumping plunger (44, 144, 244, 344) and/or the vacuum plunger (47, 147, 247, 347) comprise a matching external thread such that the pumping plunger (44, 144, 244, 344) and/or the vacuum plunger (47, 147, 247, 347) can be screwed into the first hollow cylinder (42, 142, 242, 342) and/or the second hollow cylinder (12, 112, 212, 312) in order to press the monomer liquid out of the first hollow cylinder (42, 142, 242, 342) into the internal space (24, 124, 224, 324) of the cartridge (1, 101, 201, 301) and/or to draw air out of the internal space (24, 124, 224, 324) of the cartridge (1, 101, 201, 301) into the second hollow cylinder (12, 112, 212, 312).

18. Device according to any one of the claims 1 to 16, **characterised in that**
the device comprises at least one tensioned compression spring (151) and at least one locking mechanism, whereby the compression spring (151), the vacuum plunger (47, 147, 247, 347) and/or the pumping plunger (44, 144, 244, 344) is or are locked by means of the at least one locking mechanism in detachable manner, whereby the at least one compression spring (151), with the locking mechanism detached, exerts a pressure on the pumping plunger (44, 144, 244, 344) and/or the vacuum plunger (47, 147, 247, 347) such that the pumping plunger (44, 144, 244, 344) is pressed into the first hollow cylinder (42, 142, 242, 342) and/or the vacuum plunger (47, 147, 247, 347) is pushed away from the connector (52, 152, 252, 352) to the vacuum conduit (17, 117, 217, 317) in the second hollow cylinder (12, 112, 212, 312).

19. Device according to any one of the preceding claims, **characterised in that**
the connecting conduit (38, 138, 238, 338) between the first hollow cylinder (42, 142, 242, 342) and the internal space (24, 124, 224, 324) of the cartridge (1, 101, 201, 301) comprises a loop (40, 140, 240, 340) that faces upwards, whereby the topmost point of the loop (40, 140, 240, 340) is situated above a junction (54, 154, 254, 354) of the monomer container (60, 160, 260, 360) or of the connector (20, 120, 220, 320) for the monomer container (60, 160, 260, 360) into the first hollow cylinder (42, 142, 242, 342).

20. Method for the mixing of a bone cement, in particular using a device according to any one of the claims 1 to 19, comprising the chronological steps of
A) a monomer container (60, 160, 260, 360) being opened;
B) a monomer liquid flowing from the monomer container (60, 160, 260, 360) into a first hollow cylinder (42, 142, 242, 342), whereby the first hollow cylinder (42, 142, 242, 342) is bounded on one side by a pumping plunger (44, 144, 244, 344);
C) the pumping plunger (44, 144, 244, 344) being pushed into the first hollow cylinder (42, 142, 242, 342) and the monomer liquid thus being pressed out of the first hollow cylinder (42, 142, 242, 342) and through a connecting conduit (38, 138, 238, 338) into the internal space (24, 124, 224, 324) of a cartridge (1, 101, 201, 301), whereby a cement powder is situated in the internal space (24, 124, 224, 324) of the cartridge (1, 101, 201, 301).
D) the motion of the pumping plunger (44, 144, 244, 344) moving a vacuum plunger (47, 147, 247, 347), which is connected to the pumping plunger (44, 144, 244, 344) or driven parallel to same, in a second hollow cylinder (12, 112, 212, 312) away from a connector (52, 152, 252, 352) of a vacuum conduit (17, 117, 217, 317), whereby the gas pressure between the connector (52, 152, 252, 352) of a vacuum conduit (17, 117, 217, 317) and the vacuum plunger (47, 147, 247, 347) in the second hollow cylinder (12, 112, 212, 312) is reduced due to the motion of the vacuum plunger (47, 147, 247, 347) and gas is evacuated, through the vacuum conduit (17, 117, 217, 317), from the internal space (24, 124, 224, 324) of the cartridge (1, 101, 201, 301) that is connected to the vacuum conduit (17, 117, 217, 317), and
E) the monomer liquid and the cement powder being mixed in the internal space (24, 124, 224, 324) of the cartridge (1, 101, 201, 301).

21. Method according to claim 20, **characterised in that**
the air is aspirated from the internal space (24, 124, 224, 324) of the cartridge (1, 101, 201, 301) is aspirated through the vacuum conduit (17, 117, 217, 317) on the side opposite from the junction of the connecting conduit (38, 138, 238, 338), whereby it is preferred to have the connecting conduit (38, 138, 238, 338) merge into the internal space (24, 124, 224, 324) of the cartridge (1, 101, 201, 301) on the underside of the internal space (24, 124, 224, 324) and to have the vacuum conduit (17, 117, 217, 317) merge into the internal space (24, 124, 224, 324) of the cartridge (1, 101, 201, 301) on the upper side of the internal space (24, 124, 224, 324).

22. Method according to claim 20 or 21, **characterised in that**
the monomer liquid and the cement powder are mixed in the internal space (24, 124, 224, 324) by means of a mixing facility (26, 126, 226, 326) by operating the mixing facility (26, 126, 226, 326) by moving a mixing rod (4, 104, 204, 304) that extends into the internal space (24, 124, 224, 324) of the cartridge (1, 101, 201, 301) and can be rotated and can be shifted in longitudinal direction, whereby it is preferred to pull the mixing rod (4, 104, 204, 304) out of the internal space (24, 124, 224, 324) of the cartridge (1, 101, 201, 301) up to the limit stop after the mixing and it is particularly preferred to break off the mixing rod (4, 104, 204, 304) at a predetermined breakage site after pulling it out to the limit stop.

23. Method according to any one of the claims 20 to 22, **characterised in that**
the pumping plunger (44, 144, 244, 344) is pushed into the first hollow cylinder (42, 142, 242, 342) by means of a tensioned elastic spring element (151) and/or the vacuum plunger (47, 147, 247, 347) is moved in the second hollow cylinder (12, 112, 212, 312) by means of a tensioned elastic spring element (151), whereby it is preferred to first detach at least one locking mechanism that engages or engage the pumping plunger (44, 144, 244, 344), the vacuum plunger (47, 147, 247, 347) and/or the spring element (151) for this purpose.

24. Method according to any one of the claims 20 to 23, **characterised in that**
the cartridge (1, 101, 201, 301) containing the ready-mixed cement dough is detached from the connecting conduit (38, 138, 238, 338), vacuum conduit (17, 117, 217, 317), first hollow cylinder (42, 142, 242, 342), second hollow cylinder (12, 112, 212, 312) and monomer container (60, 160, 260, 360), and the ready-mixed cement dough is dispensed from the internal space (24, 124, 224, 324) of the cartridge (1, 101, 201, 301) through propulsion of a dispensing plunger (2, 102, 202, 302), which is supported in the cartridge (1, 101, 201, 301) such as to be axially mobile and forms a boundary of the internal space (24, 124, 224, 324) of the cartridge (1, 101, 201, 301) on one side.

## Revendications

1. Dispositif de mélange d'un ciment osseux de polyméthyl méthacrylate et de stockage d'un liquide de monomère et d'une poudre de ciment servant de composants de départ du ciment osseux, le dispositif présentant
une cartouche (1, 101, 201, 301) dotée d'un espace intérieur (24, 124, 224, 324) destiné au mélange du ciment osseux, qui est fermée au niveau d'un côté par un piston d'évacuation (2, 102, 202, 302) mobile,
un récipient de monomère (60, 160, 260, 360) pour un liquide de monomère et/ou un système de raccordement (20, 120, 220, 320) pour la fixation d'un récipient de monomère (60, 160, 260, 360) destiné un liquide de monomère de sorte que le récipient de monomère (60, 160, 260, 360) peut être ouvert dans le dispositif de telle manière que le liquide de monomère s'écoule hors du récipient de monomère (60, 160, 260, 360) dans le dispositif,
une conduite de raccordement (38, 138, 238, 338) par laquelle le liquide de monomère peut être guidé dans l'espace intérieur (24, 124, 224, 324) de la cartouche (1, 101, 201, 301),
où
un premier cylindre creux (42, 142, 242, 342) est relié avec la conduite de raccordement (38, 138, 238, 338) et un deuxième cylindre creux (12, 112, 212, 312) est relié avec l'espace intérieur (24, 124, 224, 324) de la cartouche (1, 101, 201, 301) par le biais d'une conduite à vide (17, 117, 217, 317), où un piston de pompe (44, 144, 244, 344) pouvant se déplacer axialement dans le premier cylindre creux (42, 142, 242, 342) est disposé dans le premier cylindre creux (42, 142, 242, 342), et un piston à vide (47, 147, 247, 347) mobile axialement dans le deuxième cylindre creux (12, 112, 212, 312) est disposé dans le deuxième cylindre creux (12, 112, 212, 312), où le piston de pompe (44, 144, 244, 34) et le piston à vide (47, 147, 247, 347) sont mobiles simultanément, **caractérisé en ce que**
le premier cylindre creux (42, 142, 242, 342) est relié de telle manière avec le récipient de monomère (60, 160, 260, 360) et/ou le système de raccordement (20, 120, 220, 320) pour la fixation d'un récipient de monomère (60, 160, 260, 360) que le liquide de monomère s'écoule hors du récipient de monomère (60, 160, 260, 360) ouvert ou hors du récipient de monomère (60, 160, 260, 360) raccordé ouvert dans le premier cylindre creux (42, 142, 242, 342) et que la conduite de raccordement (38, 138, 238, 338) relie le premier cylindre creux (42, 142, 242, 342) avec l'espace intérieur (24, 124, 224, 32) de la cartouche (1, 101, 201, 301) de telle manière que du liquide de monomère peut être pressé avec le piston de pompe (44, 144, 244, 344) hors du premier cylindre creux (42, 142, 242, 342) par l'actionnement du piston de pompe (44, 144, 244, 34) à travers la conduite de raccordement (38, 138, 238, 338) dans l'espace intérieur (24, 124, 224, 324) de la cartouche (1, 101, 201, 301).

2. Dispositif selon la revendication 1, **caractérisé en ce que**
le piston de pompe (44, 144, 244, 344) et le piston à vide (47, 147, 247, 347) sont reliés l'un à l'autre de telle manière ou sont déplacés de telle manière par le biais d'un organe d'actionnement (315) commun, que, lors de l'insertion du piston de pompe (44, 144, 244, 344) dans le premier cylindre creux (42, 142, 242, 342), le piston à vide (47, 147, 247, 347) est déplacé dans le deuxième cylindre creux (12, 112, 212, 312) du raccordement (52, 152, 252, 352) vers la conduite à vide (17, 117, 217, 317), où, de préférence, ce faisant, le volume entre le piston à vide (47, 147, 247, 347) et le raccordement (52, 152, 252, 352) s'agrandit vers la conduite à vide (17, 117, 217, 317) dans le deuxième cylindre creux (12, 112, 212, 312), de sorte que, par la dépression générée dans le deuxième cylindre creux (12, 112, 212, 312), un gaz s'écoule hors de l'espace intérieur (24, 124, 224, 324) de la cartouche (1, 101, 201, 301) à travers la conduite à vide (17, 117, 217, 317) dans le deuxième cylindre creux (12, 112, 212, 312).

3. Dispositif selon une des revendications précédentes, **caractérisé en ce que**
le premier cylindre creux (42, 142, 242, 342) est disposé entre le récipient de monomère (60, 160, 260, 360) ou le système de raccordement (20, 120, 220, 320) pour le récipient de monomère (60, 160, 260, 360) et l'espace intérieur (24, 124, 224, 324) de la cartouche (1, 101, 201, 301), de préférence dans la conduite de raccordement (38, 138, 238, 338) entre le récipient de monomère (60, 160, 260, 360) ou le système de raccordement (20, 120, 220, 320) pour le récipient de monomère (60, 160, 260), 360) et l'espace intérieur (24, 124, 224, 324) de la cartouche (1, 101, 201, 301).

4. Dispositif selon une des revendications précédentes, **caractérisé en ce que**
le piston de pompe (44, 144, 244, 344) et le premier cylindre creux (42, 142, 242, 342) présentent une surface de section transversale plus petite que le piston à vide (47, 147, 247, 347) et le deuxième cylindre creux (12, 112, 212, 312), de préférence, le piston à vide (47, 147, 247, 347) et le deuxième cylindre creux (12, 112, 212, 312) présentent une surface de section transversale au moins égale au double de celle du piston de pompe (44, 144, 244, 344) et du premier cylindre creux (42, 142, 242, 342).

5. Dispositif selon une des revendications précédentes, **caractérisé en ce que**
le piston de pompe (44, 144, 244, 344) et le piston à vide (47, 147, 247, 347) sont reliés l'un à l'autre, de préférence sont reliés solidement l'un à l'autre, de manière particulièrement préférée, sont construits d'un seul tenant.

6. Dispositif selon la revendication 5, **caractérisé en ce que**
le côté du piston à vide (47, 147, 247) orienté vers la conduite à vide (17, 117, 217) est disposé sur le côté arrière du piston de pompe (44, 144, 244) et de manière correspondante, le côté du piston de pompe (44, 144, 244) orienté vers la conduite de raccordement (38, 138, 238) est disposé sur le côté arrière du piston à vide (47, 147, 247).

7. Dispositif selon une des revendications précédentes, **caractérisé en ce que**
la conduite à vide (17, 117, 217, 317) est reliée avec l'espace intérieur (24, 124, 224, 324) de la cartouche (1, 101, 201, 301) par le piston d'évacuation (2, 102, 202, 302), où, de préférence, un filtre (328) ou un tamis est disposé dans le piston d'évacuation (2, 102, 202, 302) par lequel la conduite à vide (17, 117, 217, 317) est reliée avec l'espace intérieur (24, 124, 224, 324) de la cartouche (1, 101, 201, 301).

8. Dispositif selon une des revendications précédentes, **caractérisé en ce que**
le récipient de monomère (60, 160, 260, 360) est disposé ou est à disposer dans un récipient en ampoule (21, 121, 221) souple, où, de préférence, au moins un orifice de ventilation (223) est prévu dans le récipient en ampoule (21, 121, 221).

9. Dispositif selon une des revendications précédentes, **caractérisé en ce**
**qu'**un dispositif de mélange (26, 126, 226, 326) qui peut être actionné de l'extérieur est disposé dans la cartouche (1, 101, 201, 301), où, de préférence, le dispositif de mélange (26, 126, 226, 326) peut être utilisé par le biais d'une tige de mélange (4, 104, 204, 304) qui est menée à travers un passage dans le piston d'évacuation (2, 102, 202, 302) vers l'intérieur de la cartouche (1, 101, 201, 301) et y est logée en étant mobile.

10. Dispositif selon une des revendications précédentes, **caractérisé en ce que**
la poudre de ciment est contenue dans l'espace intérieur (24, 124, 224, 324) de la cartouche (1, 101, 201, 301).

11. Dispositif selon une des revendications précédentes, **caractérisé en ce**
**qu'**un filtre (32, 132, 232, 332) ne laissant pas passer la poudre de ciment et perméable au liquide de monomère est disposé entre la conduite de raccordement (38, 138, 238, 338) et l'espace intérieur (24, 124, 224, 324) de la cartouche (1, 101, 201, 301).

12. Dispositif selon une des revendications précédentes, **caractérisé en ce que**
le dispositif présente un pied support (10, 110, 210) dans lequel une partie de la conduite de raccordement (38, 138, 238, 338) est disposée, où la cartouche (1, 101, 201, 301) est reliée de manière amovible avec le pied support (10, 110, 201), notamment de manière amovible avec le pied support (10, 110, 210) par le biais d'un filetage à visser, où, de préférence, le filtre (32, 132, 232, 332) ne laissant pas passer la poudre de ciment et perméable au liquide de monomère est éventuellement disposé dans le pied support (10, 110, 210) du dispositif, de manière particulièrement préférée, est disposé dans le raccordement (34, 134, 234) vers la cartouche (1, 101, 201, 301) du pied support (10, 110, 201).

13. Dispositif selon la revendication 12, **caractérisé en ce que**
le premier cylindre creux (42, 142, 242, 342), le deuxième cylindre creux (12, 112, 212, 312) et le récipient de monomère (60, 160, 260, 360), ou le premier cylindre creux (42, 142, 242, 342), le deuxième cylindre creux (12, 112, 212, 312) et le système de raccordement (20, 120, 220, 320) sont reliés, de préférence de manière inamovible, avec le pied support (10, 110, 210) pour la fixation du récipient de monomère (60, 160, 260, 360) avec le pied support (10, 110, 210).

14. Dispositif selon une des revendications précédentes, **caractérisé en ce que**
le récipient de monomère (60, 160, 260, 360) pour le liquide de monomère, ou le système de raccordement (20, 120, 220, 320) pour la fixation du récipient de monomère (60, 160, 260, 360), débouche au niveau d'une surface d'enveloppe du premier cylindre creux (42, 142, 242, 342) dans le premier cylindre creux (42, 142, 242, 342), de préférence directement en-dessous du piston de pompe (44, 144, 244, 344) dans le premier cylindre creux (42, 142, 242, 342).

15. Dispositif selon une des revendications précédentes, **caractérisé en ce que**
le récipient de monomère (60, 160, 260, 360) est disposé au-dessus du raccordement (54, 154, 254, 354) par rapport au premier cylindre creux (42, 142, 242, 342).

16. Dispositif selon une des revendications précédentes, **caractérisé en ce que**
le piston de pompe (44, 144, 244, 344) peut être actionné manuellement axialement dans le premier cylindre creux (42, 142, 242, 342), de préférence, peut être pressé manuellement axialement à l'intérieur du premier cylindre creux (42, 142, 242, 342), et/ou le piston à vide (47, 147, 247, 347) peut être déplacé manuellement axialement dans le deuxième cylindre creux (12, 112, 212, 312).

17. Dispositif selon une des revendications précédentes, **caractérisé en ce que**
le premier cylindre creux (42, 142, 242, 342), et/ou le deuxième cylindre creux (12, 112, 212, 312), présente un filetage intérieur et le piston de pompe (44, 144, 244, 344), et/ou le piston à vide (47, 147, 247, 347), présente un filetage extérieur lui correspondant, de sorte que le piston de pompe, et/ou le piston à vide (47, 147, 247, 347), peut être vissé dans le premier cylindre creux (42, 142, 242, 342), et/ou le deuxième cylindre creux (12, 112, 212, 312), afin de presser le liquide de monomère hors du premier cylindre creux (42, 142, 242, 342) dans l'espace intérieur (24, 124, 224, 324) de la cartouche (1, 101, 201, 301) et/ou afin d'aspirer de l'air hors de l'espace intérieur (24, 124, 224, 324) de la cartouche (1, 101, 201, 301) dans le deuxième cylindre creux (12, 112, 212, 312).

18. Dispositif selon une des revendications 1 à 16, **caractérisé en ce que**
le dispositif présente au moins un ressort de compression (151) contraint et au moins une butée, où le ressort de compression (151), le piston à vide (47, 147, 247, 347) et/ou le piston de pompe (44, 144, 244, 344) peut ou peuvent être mis en butée de manière amovible avec l'au moins une butée, où, pour une butée hors service, l'au moins un ressort de compression (151) exerce une pression sur le piston de pompe (44, 144, 244, 344), et/ou le piston à vide (47, 147, 247, 347), de sorte que le piston de pompe (44, 144, 244, 344) est pressé dans le premier cylindre creux (42, 142, 242, 342), et/ou le piston à vide (47, 147, 247, 347) est pressé par le système de raccordement (52, 152, 252, 352), vers la conduite à vide (17, 117, 217, 317) en s'éloignant dans le deuxième cylindre creux (12, 112, 212, 312).

19. Dispositif selon une des revendications précédentes, **caractérisé en ce que**
la conduite de raccordement (38, 138, 238, 338) présente une boucle (40, 140, 240, 340) orientée vers le haut entre le premier cylindre creux (42, 142, 242, 342) et l'espace intérieur (24, 124, 224, 324) de la cartouche (1, 101, 201, 301), où le point le plus haut de la boucle (40, 140, 240, 340) se situe au-dessus d'une embouchure (54, 154, 254, 354) du récipient de monomère (60, 160, 260, 360) ou du système de raccordement (20, 120, 220, 320) pour le récipient de monomère (60, 160, 260, 360) dans le premier cylindre creux (42, 142, 242, 342).

20. Procédé de mélange d'un ciment osseux, notamment avec un dispositif selon l'une des revendications 1 à 19, avec les étapes chronologiques suivantes
A) un récipient de monomère (60, 160, 260, 360) est ouvert,
B) un liquide de monomère s'écoule hors du récipient de monomère (60, 160, 260, 360) dans un premier cylindre creux (42, 142, 242, 342), où le premier cylindre creux (42, 142, 242, 342) est délimité sur un côté par un piston de pompe (44, 144, 244, 344),
C) le piston de pompe (44, 144, 244, 344) est pressé à l'intérieur du premier cylindre creux (42, 142, 242, 342) et le liquide de monomère est ainsi pressé hors du premier cylindre creux (42, 142, 242, 342) et à travers une conduite de raccordement (38, 138, 238, 338) dans l'espace intérieur (24, 124, 242, 342) d'une cartouche (1, 101, 201, 301), où une poudre de ciment se trouve dans l'espace intérieur (24, 124, 224, 324) de la cartouche (1, 101, 201, 301),
D) un piston à vide (47, 147, 247, 347) relié avec le piston de pompe (44, 144, 244, 344) ou un piston à vide (47, 147, 247, 347) entraîné parallèlement est déplacé avec le déplacement du piston de pompe (44, 144, 244, 344) dans un deuxième cylindre creux (12, 112, 212, 312) par un système de raccordement (52, 152, 252, 352) d'une conduite à vide (17, 117, 217, 317), où la pression de gaz entre le système de raccordement (52, 152, 252, 352) de la conduite à vide (17, 117, 217, 317) et le piston à vide (47, 147, 247, 347) dans le deuxième cylindre creux (12, 112, 212, 312) est diminuée par le déplacement du piston à vide (47, 147, 247, 347) et le gaz est évacué par la conduite à vide (17, 117, 217, 317) hors de l'espace intérieur (24, 124, 224, 324) de la cartouche (1, 101, 201, 301) raccordé sur la conduite à vide (17, 117, 217, 317), et
E) le liquide de monomère et la poudre de ciment sont mélangés dans l'espace intérieur (24, 124, 224, 324) de la cartouche (1, 101, 201, 301).

21. Procédé selon la revendication 20, **caractérisé en ce que**
l'air est aspiré hors de l'espace intérieur (24, 124, 224, 324) de la cartouche (1, 101, 201, 301) sur le côté se situant en face de l'embouchure de la conduite de raccordement (38, 138, 238, 338) à travers la conduite à vide (17, 117, 217, 317), où, de préférence, la conduite de raccordement (38, 138, 238, 338) débouche dans l'espace intérieur (24, 124, 224, 324) de la cartouche (1, 101, 201, 301) au niveau du côté inférieur de l'espace intérieur (24, 124, 224, 324) et la conduite à vide (17, 117, 217, 317) débouche dans l'espace intérieur (24, 124, 224, 324) de la cartouche (1, 101, 201, 301) au niveau du côté supérieur de l'espace intérieur (24, 124, 224, 324).

22. Procédé selon la revendication 20 ou la revendication 21, **caractérisé en ce que**
le liquide de monomère et la poudre de ciment sont mélangés dans l'espace intérieur (24, 124, 224, 324) avec un dispositif de mélange (26, 126, 226, 326), **en ce que** le dispositif de mélange (26, 126, 226, 326) est actionné par le déplacement d'une tige de mélange (4, 104, 204, 304) menée en pouvant tourner et en pouvant se déplacer dans la direction longitudinale dans l'espace intérieur (24, 124, 224, 324) de la cartouche (1, 101, 201, 301), où, de préférence, après le mélange, la tige de mélange (4, 104, 204, 304) est retirée de l'espace intérieur (24, 124, 224, 324) de la cartouche (1, 101, 201, 301) jusqu'à la butée, et de manière particulièrement préférée, la tige de mélange (4, 104, 204, 304) est cassée au niveau d'un point de rupture souhaité après le retrait jusqu'à la butée.

23. Procédé selon l'une des revendications 20 à 22, **caractérisé en ce que**
le piston de pompe (44, 144, 244, 344) est pressé à l'intérieur du premier cylindre creux (42, 142, 242, 342) avec un élément ressort (151) élastique contraint et/ou le piston à vide (47, 147, 247, 347) est déplacé dans le deuxième cylindre creux (12, 112, 212, 312) avec un élément ressort (151) élastique contraint, où, à cette fin, de préférence, au moins une butée est auparavant mise hors service, qui est ou sont en prise dans le piston de pompe (44, 144, 244, 344), le piston à vide (47, 147, 247, 347) et/ou l'élément ressort (151).

24. Procédé selon l'une des revendications 20 à 23, **caractérisé en ce que**
la cartouche (1, 101, 201, 301) avec la pâte de ciment mélangée terminée est détachée de la conduite de raccordement (38, 138, 238, 338), de la conduite à vide (17, 117, 217, 317), du premier cylindre creux (42, 142, 242, 342), du deuxième cylindre creux (12, 112, 212, 312) et du récipient de monomère (60, 160, 260, 360), et la pâte de ciment mélangée terminée est évacuée hors de l'espace intérieur (24, 124, 224, 324) de la cartouche (1, 101, 201, 301) par la poussée d'un piston d'évacuation (2, 102, 202, 302) qui est logé en étant mobile axialement dans la cartouche (1, 101, 201, 301) et délimite l'espace intérieur (24, 124, 224, 324) de la cartouche (1, 101, 201, 301) sur un côté.
